# EUROPÄISCHE PATENTSCHRIFT

(11) **EP 3 743 144 B1**
(45) Veröffentlichungstag und Bekanntmachung des Hinweises auf die Patenterteilung: **18.01.2023**
(21) Anmeldenummer: 19742725.5
(22) Anmeldetag: 19.07.2019
(51) Int. Cl.: A61M 16/18

(54) **VERBINDUNGS-ANORDNUNG ZUM VERBINDEN EINES NARKOSEMITTEL-BEHÄLTERS MIT EINEM NARKOSEMITTEL-VERDAMPFER**
CONNECTION ARRANGEMENT FOR CONNECTING AN ANAESTHETIC CONTAINER TO AN ANAESTHETIC VAPORIZER
ENSEMBLE DE LIAISON POUR RELIER UN RÉCIPIENT CONTENANT UN ANESTHÉSIQUE À UN ÉVAPORATEUR D'ANESTHÉSIQUE

(30) Priorität: 08.08.2018 DE 102018006264
(43) Veröffentlichungstag der Anmeldung: 02.12.2020
(73) Patentinhaber: Drägerwerk AG & Co. KGaA, 23558 Lübeck (DE)
(72) Erfinder: ZECHLIN, Jacob-Michael, 23562 Lübeck (DE); BUCHHOLZ, Moritz, 22303 Hamburg (DE); PRIOR, Daisy, Cambridge CB1 3DW (GB); FIELDING, Lucy, Histon CB24 9YR (GB); KRÖPLIN, Thomas, 23701 Süsel (DE); HAMLIN, Frederick William, 02111 Boston (US); MAUCHLE, Alexander Roland, CB4 0DW Cambridge (GB)
(86) Internationale Anmeldenummer: PCT/EP2019/069487
(87) Internationale Veröffentlichungsnummer: WO 2020/030408

(56) Entgegenhaltungen:
- WO-A1-2005/056093
- WO-A1-2008/151668
- GB-A- 2 531 253

## Beschreibung

Die Erfindung betrifft eine Verbindungs-Anordnung, welche einen Adapter eines Narkosemittel-Behälters lösbar mit einem Anschlussabschnitt eines Narkosemittel-Verdampfers zu verbinden vermag und dadurch ermöglicht, dass Narkosemittel aus dem Narkosemittel-Behälter in den Narkosemittel-Verdampfer fließt, sowie einen Narkosemittel-Verdampfer und einen Narkosemittel-Adapter.

Ein Anästhesiegerät versorgt einen Patienten mit einem Gemisch aus Luft oder einem anderen Gas und verdampftem Narkosemittel. Ein zuvor flüssiges Narkosemittel wird in einem Narkosemittel-Verdampfer verdampft, indem es auf z.B. ca. 40 °C erhitzt wird, und mit dem Gas vermischt. Ein solcher Narkosemittel-Verdampfer umfasst in der Regel einen Narkosemittel-Tank. Von Zeit zu Zeit muss dieser Narkosemittel-Tank aufgefüllt werden. Das flüssige Narkosemittel wird in der Regel in einem Narkosemittel-Behälter zu dem Anästhesiegerät mit dem Narkosemittel-Verdampfer transportiert. Dort wird eine in der Regel lösbare Fluidverbindung zwischen dem Narkosemittel-Behälter und dem Narkosemittel-Verdampfer hergestellt. Der Fluidverbindung verläuft in der Regel vom Narkosemittel-Behälter schräg nach unten in den Narkosemittel-Verdampfer. Nachdem die Fluidverbindung hergestellt ist, fließt das flüssige Narkosemittel durch diese Fluidverbindung aus dem Narkosemittel-Behälter in den Narkosemittel-Tank des Narkosemittel-Verdampfers.

Verschiedene Ausgestaltungen, um einen Narkosemittel-Verdampfer mit Narkosemittel zu befüllen, sind bekannt geworden.

In DE 10 2004 043 652 B3 wird eine Füllvorrichtung für einen Narkosemittel-Verdampfer beschrieben, welche zur Aufnahme von Anschlussmitteln einer Narkosemittelflasche ausgebildet ist und ein Füllventil umfasst, welches dazu bestimmt ist, nach Bedarf zu öffnen oder zu schließen, wobei die Narkosemittel-Flasche und ein Narkosemittel-Tank des Narkosemittel-Verdampfers während eines Befüllvorgangs in Form von kommunizierenden Behältern über einen vom Flüssigkeitsspiegel des Narkosemittels im Narkosemittel-Tank verschließbaren Fluidkanal miteinander verbunden sind. Ferner ist eine oberhalb des Fluidkanals liegende, im Bedarfsfall offenbare oder verschließbare Entlüftungsbohrung vorgesehen, durch welche ein zusätzliches, über dem Flüssigkeitsniveau des Fluidkanals liegendes Narkosemittel-Volumen in den Narkosemittel-Tank einfüllbar ist.

US 2004/0206417 A1 zeigt einen Narkosemittel-Verdampfer (vaporizer 9) mit einem Narkosemittel-Tank (internal sump) und einer Auffüllstation (filling station 98). Auf eine Flasche mit Narkosemittel (anesthetic bottle 8) lässt sich ein Adapter (bottle adapter 10) einsetzen. Der Adapter 10 besitzt ein Adapterventil (adapter valve assembly 66) mit einem Schaft (elongated valve stem 68), den eine Feder (bias spring 84) von der Narkosemittel-Flasche 8 weggedrückt. Die Auffüllstation 98 besitzt eine stationäre Aktivierungsstange (stationary activation rod 160). Der Adapter 10 lässt sich in die Auffüllstation 98 einführen. Die Aktivierungsstange 160 drückt den Schaft 68 gegen die Kraft der Feder 84 in den Adapter 10 hinein, wodurch eine Passage aus der Flasche 8 in den Narkosemittel-Tank geöffnet wird. WO 2011/070591 A2 zeigt einen Adapter 50, der sich mit einem Narkosemittel-Behälter (Container) verbinden lässt, sowie einen Narkosemittel-Verdampfer, der mit einem Anschlussabschnitt (receiving head, a receiver subassembly 30 attached to the vaporizer) verbunden oder verbindbar ist. Der Behälter lässt sich durch eine zweistufige Bewegung mit dem Verdampfer verbinden: Zunächst wird der Adapter 50 linear zum Anschlussabschnitt 30 hin geschoben und anschließend gedreht.

In US 2007/0199616 A1 wird ein Adapter 10 beschrieben, der sich mit einem Behälter (liquid anesthetic agent container or bottle 18) für Narkosemittel verbinden lässt und eine Ventil-Anordnung (movable adapter valve assembly 16) aufweist. Dieser Adapter 10 lässt sich in einen Anschluss (anesthetic vaporizer inlet port 68) eines Verdampfers einsetzen. Ein radialer Vorsprung (radial ledge 82) im Anschluss 68 umgibt einen Kontaktstift 74. Wenn der Adapter 10 in den Anschluss 68 eingesetzt ist, so kommt der radiale Vorsprung 82 in Kontakt mit vier gebogenen Verbindungselementen 44, welche die Ventilanordnung 16 öffnen.

US 2013/0098498 A1 zeigt eine Vorrichtung (device 10), um einen Narkosemittel-Behälter (anesthetic reservoir 70) mit einem Narkosemittel-Verdampfer (vaporizer) zu verbinden. Ein Stempel (plunger 53) verschließt eine Öffnung (outlet 57) in einem Anschlussstück (vaporizer receiving port 52) des Verdampfers, solange der Verdampfer nicht mit dem Behälter 70 verbunden ist. Die Vorrichtung 10 ist mit dem Behälter 70 verbunden und umfasst eine erste Komponente 12, die sich fluiddicht mit einer Öffnung 72 des Behälters 10 verbinden lässt, und eine schmalere zweite Komponente 20, welche sich in das Anschlussstück 52 hineinschieben lässt. Wenn die Vorrichtung 10 in das Anschlussstück 52 eingeschoben wird, drückt eine zum Verdampfer hin zeigende Seite (downstream side) eines Stößels (support member 34) den Stempel 53 in den Verdampfer hinein, so dass die Öffnung 57 freigegeben wird und Narkosemittel in den Verdampfer fließen kann.

Der Erfindung liegt die Aufgabe zugrunde, eine Verbindungs-Anordnung zum lösbaren Verbinden eines Narkosemittel-Behälters mit einem Narkosemittel-Verdampfer bereitzustellen, welche es ermöglicht, mit einer größeren Betriebssicherheit als bekannte Verbindungs-Anordnungen einen Narkosemittel-Verdampfer mit Narkosemittel aus einem Narkosemittel-Behälter zu befüllen.

Die Aufgabe wird durch eine Verbindungs-Anordnung mit den Merkmalen des Anspruchs 1 und durch einen Anschlussabschnitt gemäß Anspruch 14 für einen Narkosemittel-Verdampfer gelöst.

Die erfindungsgemäße Verbindungs-Anordnung ist dazu ausgestaltet, einen Narkosemittel-Behälter zeitweise mit einem Narkosemittel-Verdampfer fluiddicht zu verbinden.

Die Verbindungs-Anordnung umfasst einen Anschlussabschnitt und einen Adapter. Der Anschlussabschnitt ist mit dem Narkosemittel-Verdampfer verbunden oder lässt sich wenigstens zeitweise mit ihm verbinden. Der Adapter ist mit dem Narkosemittel-Behälter verbunden oder lässt sich wenigstens zeitweise mit ihm verbinden.

Der Adapter lässt sich lösbar mit dem Anschlussabschnitt verbinden. Die Verbindung lässt sich also wieder lösen.

Der Anschlussabschnitt umfasst einen verdampferseitigen Kanalabschnitt. Der Adapter umfasst einen behälterseitigen Kanalabschnitt. Dann, wenn der Adapter mit dem Anschlussabschnitt verbunden ist, bilden der verdampferseitige und der behälterseitige Kanalabschnitt zusammen einen durchgehenden Kanal. Mithilfe dieses durchgehenden Kanals ist eine Fluidverbindung zwischen dem Narkosemittel-Behälter und dem Narkosemittel-Verdampfer hergestellt. Oder eine solche Fluidverbindung lässt sich herstellen.

Wenn der Adapter mit dem Anschlussabschnitt verbunden ist, wird derjenige Bereich der Oberfläche des Anschlussabschnitts, der dann zum Adapter hin zeigt, von einem verdampferseitigen Kontaktprofil gebildet. Derjenige Bereich der Oberfläche des Adapters, der dann zum Anschlussabschnitt hin zeigt, wird von einem behälterseitigen Kontaktprofil gebildet. Mit anderen Worten: Die beiden Oberflächen-Bereiche stellen diese beiden Kontaktprofile bereit. Diese beiden Kontaktprofile liegen ohne Zwischenraum aneinander an, wenn der Adapter mit dem Anschlussabschnitt verbunden ist. "Ohne Zwischenraum" bedeutet: Das Volumen eines solchen Zwischenraums ist vernachlässigbar klein, kann also keine relevante Menge eines Fluids aufnehmen.

Das eine Kontaktprofil, also entweder das verdampferseitige Kontaktprofil oder das behälterseitige Kontaktprofil, weist einen Vorsprung auf. Das andere Kontaktprofil, also das behälterseitige Kontaktprofil oder das verdampferseitige Kontaktprofil, weist eine korrespondierende Aussparung auf. Wenn der Adapter mit dem Anschlussabschnitt verbunden ist, greift der Vorsprung des einen Kontaktprofils formschlüssig in die Aussparung des anderen Kontaktprofils ein. Die erfindungsgemäße Verbindungs-Anordnung ermöglicht es und lässt sich dafür verwenden, eine lösbare Fluidverbindung zwischen einem Narkosemittel-Behälter und einem Narkosemittel-Verdampfer herzustellen. Dadurch wird ermöglicht, einen Narkosemittel-Tank des Narkosemittel-Verdampfers aufzufüllen. Die Fluidverbindung lässt sich wieder lösen, sodass der Narkosemittel-Behälter nach dem Lösen der Fluidverbindung nicht den Einsatz des Narkosemittel-Verdampfers behindert und mit weiterem Narkosemittel aufgefüllt oder entsorgt werden kann. Möglich, aber nicht erforderlich ist, dass der Narkosemittel-Verdampfer dauerhaft in einer Fluidverbindung mit einem externen Reservoir für Narkosemittel steht. Der verdampferseitige Kanalabschnitt und der behälterseitige Kanalabschnitt bilden dann, wenn der Adapter mit dem Anschlussabschnitt verbunden ist, einen durchgehenden Kanal. Durch diesen Kanal lässt sich ein Narkosemittel-Tank des Narkosemittel-Verdampfers mit Narkosemittel aus dem Narkosemittel-Behälter auffüllen. Möglich ist, dass die Fluidverbindung immer dann gebildet wird, wenn der Adapter mit dem Anschlussabschnitt verbunden ist und der durchgehende Kanal hergestellt ist. Möglich ist auch, dass zusätzlich noch eine Sperre geöffnet werden muss, insbesondere gegen die Kraft einer Feder, um die Fluidverbindung zwischen dem Narkosemittel-Behälter und dem Narkosemittel-Verdampfer herzustellen.

Erfindungsgemäß liegen die beiden Kontaktprofile dann, wenn der Adapter mit dem Anschlussabschnitt verbunden ist, ohne Zwischenraum aneinander an. Die beiden Kontaktprofile nehmen nicht notwendigerweise den gesamten Platz zwischen dem Aufnahmeabschnitt und dem Adapter an. Falls aufgrund eines Abstandes zwischen dem Aufnahmeabschnitt und dem Adapter mindestens ein Zwischenraum gebildet wird, so steht dieser Zwischenraum in Fluidverbindung mit dem verdampferseitigen Kanalabschnitt. Erfindungsgemäß sind die Kontaktprofile so ausgestaltet, dass kein abgeschlossener Zwischenraum mit einem relevanten Volumen zwischen dem Aufnahmeabschnitt und dem Adapter gebildet wird. Durch dieses erfindungsgemäße Merkmal wird verhindert, dass sich zwischen den beiden Kontaktprofilen in einem abgeschlossenen Zwischenraum ein großer Totraum bilden kann. In einem solchen Totraum zwischen den beiden Kontaktprofilen kann sich nämlich während eines Befüllvorgangs flüssiges Narkosemittel sammeln. Sobald der Adapter wieder vom Anschlussabschnitt getrennt wird, weil der Füllvorgang beendet ist, wird der Totraum geöffnet, und das Narkosemittel im Totraum ist der Umgebung ausgesetzt. Typischerweise verdampft Narkosemittel bereits bei Zimmertemperatur, so dass bei einem großen Totraum nach dem Trennen schlagartig eine relevante Menge von Narkosemittel verdampft und gasförmiges Narkosemittel in die Umgebung entweicht. Das oft verwendete Narkosemittel Desfluran hat einen Siedepunkt von etwa 23 °C. Der Austritt von Narkosemittel in die Umgebung ist unerwünscht. Die Erfindung verhindert dieses unerwünschte Ereignis.

Weil die Kontaktflächen ohne Zwischenraum aneinander anliegen, wird in vielen Fällen auch keine Dichtung benötigt, um einen solchen Zwischenraum gegen Narkosemittel abzudichten. Dies ist insbesondere deshalb von Vorteil, weil ein Kontakt eines flüssigen Narkosemittels mit einer Dichtung dazu führen kann, dass die Dichtung angegriffen wird oder aufquillt, und eine Dichtung somit durch Narkosemittel beschädigt werden kann. Gegen Narkosemittel resistente Dichtungsmittel sind oft teuer. Außerdem übt eine Dichtung aus elastischem Material eine Kraft aus, die einer Bewegung des Adapters relativ zum Anschlussabschnitt entgegengesetzt ist. Dieser Effekt ist manchmal unerwünscht, weil diese Kraft mit einem Kraftaufwand überwunden werden muss. Die erfindungsgemäße Verbindungs-Anordnung kann aber auch eine Dichtung umfassen.

Erfindungsgemäß greift ein Vorsprung des einen Kontaktprofils formschlüssig in die korrespondierende Aussparung des anderen Kontaktprofils ein. Dadurch wird sichergestellt, dass der Adapter nach dem Verbinden mit dem Anschlussabschnitt in einer korrekten Position relativ zu dem Anschlussabschnitt sitzt. Oft wird ermöglicht, dass der Adapter geführt wird, während er auf den Anschlussabschnitt zu bewegt wird. Weiterhin wird verhindert, dass der Adapter sich relativ zum Anschlussabschnitt seitlich verschieben lässt. Der Begriff "seitlich" bezieht sich auf eine Verschieberichtung, in welcher der Adapter verschoben wird, um mit dem Anschlussabschnitt verbunden zu werden.

Erfindungsgemäß umfasst der Anschlussabschnitt ein hohles äußeres verdampferseitiges Bauteil und ein inneres verdampferseitiges Bauteil. Der verdampferseitige Kanalabschnitt ist durch das innere verdampferseitige Bauteil hindurch geführt. Das innere verdampferseitige Bauteil und somit der verdampferseitige Kanalabschnitt sind durch das äußere verdampferseitige Bauteil hindurch geführt. Der verdampferseitige Kanalabschnitt ist daher durch zwei überlappende Bauteile vor der Umgebung geschützt. Diese Ausgestaltung verringert weiter die Gefahr, dass ein Leck auftritt und Narkosemittel aus dem verdampferseitigen Kanalabschnitt austritt.

Bevorzugt lässt sich das äußere verdampferseitige Bauteil relativ zum inneren verdampferseitigen Bauteil bewegen, besonders bevorzugt zwischen einer Sperr-Endposition, in welcher der verdampferseitige Kanalabschnitt gesperrt ist, und einer Freigabe-Endposition, in welcher dieser geöffnet ist.

In einer Weiterbildung dieser Ausgestaltung umfasst das äußere verdampferseitige Bauteil ein Stellmittel. Das Stellmittel vermag in einer Sperr-Endposition den verdampferseitigen Kanalabschnitt zu versperren und in einer Freigabe-Endposition den verdampferseitigen Kanalabschnitt freizugeben. Weil dieses Stellmittel zum äußeren und nicht zum inneren verdampferseitigen Bauteil gehört, beeinträchtigt das Stellmittel in der Freigabe-Endposition nicht den Fluss eines Narkosemittel durch den verdampferseitigen Kanalabschnitt im inneren verdampferseitigen Bauteil. In der Sperr-Endposition verhindert das Stellmittel, dass Narkosemittel austritt. Bevorzugt gibt das Stellmittel den verdampferseitigen Kanalabschnitt frei, sobald das Stellmittel auf dem Weg von der Sperr-Endposition in die Freigabe-Endposition eine bestimmte Zwischen-Position erreicht hat. Bevorzugt ist das Stellmittel hohl, umschließt also einen Innenraum. Das innere verdampferseitige Bauteil ist in dieser Ausgestaltung durch das Stellmittel hindurch geführt. Besonders bevorzugt sind das Stellmittel und optional ein weiteres, bevorzugt ebenfalls hohles, Element des äußeren verdampferseitigen Bauteils einteilig ausgebildet und umgeben wenigstens teilweise das innere verdampferseitige Bauteil. Diese Ausgestaltung führt zu einer besonders robusten und mechanisch einfachen Bauweise. Ein seitlich überstehendes Teil, insbesondere ein Hebel, ist nicht erforderlich. Ein solches überstehende Teil kann zu einer Verletzung einer Person führen oder beschädigt werden.

Bevorzugt umfasst der Anschlussabschnitt ein verdampferseitiges Federelement. Dieses Federelement stützt sich bevorzugt am inneren verdampferseitigen Bauteil ab, besonders bevorzugt an einem umlaufenden Vorsprung dieses inneren Bauteils. Das verdampferseitige Federelement ist bestrebt, das Stellmittel in die Sperr-Endposition zu bewegen und in dieser zu halten. Bevorzugt ist das verdampferseitige Federelement bestrebt, das Stellmittel vom Narkosemittel-Verdampfer weg und auf den Adapter zu zu bewegen. Das verdampferseitige Federelement trägt daher dazu bei, den verdampferseitigen Kanalabschnitt zu verschließen und verschlossen zu halten. Um das Stellmittel in die Freigabe-Endposition oder optional wenigstens in eine Zwischen-Position zu verbringen, in welche ein Fluss von Narkosemittel ermöglicht wird, muss das Stellmittel gegen die Kraft des verdampferseitigen Federelements bewegt werden. Diese Ausgestaltung reduziert weiter die Gefahr, dass Narkosemittel ungewollt aus dem Anschlussabschnitt oder aus dem verbundenen Narkosemittel-Verdampfer austritt. Besonders bevorzugt ist das verdampferseitige Federelement hohl, ist beispielsweise eine Schraubenfeder, und ein Teil des inneren verdampferseitigen Bauteils, durch welches der verdampferseitige Kanalabschnitt geführt ist, ist durch dieses hohle Federelement hindurch geführt. Dadurch beeinträchtigt das verdampferseitige Federelement nicht den Fluss von Fluid durch den verdampferseitigen Kanalabschnitt.

In einer Ausgestaltung ist das innere verdampferseitige Bauteil nach Art eines Pilzes aufgebaut oder umfasst einen pilzförmigen Teil, d. h. das innere verdampferseitige Bauteil umfasst einen Kopf und eine Röhre. Der Kopf hat einen größeren maximalen Durchmesser als die Röhre. Der verdampferseitige Kanalabschnitt ist durch die Röhre hindurch oder um die Röhre herum geführt. In einer Ausgestaltung sitzt der Kopf direkt auf der Röhre, in einer anderen Ausgestaltung verbindet ein Hals, der schmaler als der Kopf ist, den Kopf mit der Röhre. Möglich ist, dass der verdampferseitige Kanalabschnitt durch die Röhre hindurch und am Hals vorbei geführt ist.

Wenn das Stellmittel in der Sperr-Endposition ist, liegt das Stellmittel bevorzugt an dem Kopf an. Der Kopf fungiert also als ein Anschlagelement für das Stellmittel. Umgekehrt bewirkt die Ausübung einer Kraft auf das Stellmittel, dass das Stellmittel vom Kopf weg bewegt wird. In einer Ausgestaltung ist das Stellmittel in der Sperr-Endposition, wenn es am Kopf anliegt, und in der Freigabe-Endposition, wenn es vom Kopf weg bewegt ist und einen Abstand zum Kopf aufweist, besonders bevorzugt den maximal möglichen Abstand. Bevorzugt ist das verdampferseitige Federelement bestrebt, das Stellmittel gegen den Kopf zu drücken. Bevorzugt umgibt das Stellmittel die Röhre.

In einer Ausgestaltung ist in das Stellmittel mindestens eine Öffnung eingelassen, die zum verdampferseitigen Kanalabschnitt gehört. Wenn das Stellmittel in der Sperr-Endposition ist, versperrt der Kopf die oder jede Öffnung im Stellmittel, so dass das Stellmittel die Sperrwirkung ausübt und den verdampferseitigen Kanalabschnitt versperrt.

Bevorzugt bildet ein Segment des Kopfes eine Hinterschneidung aus. Bevorzugt liegt am Kopf ein Dichtring an, der die Röhre umgibt und dann, wenn das Stellmittel in der Sperr-Endposition ist, die oder jede Öffnung im Stellmittel und damit den verdampferseitigen Kanalabschnitt verschließt.

In einer Ausgestaltung ist sowohl im inneren verdampferseitigen Bauteil als auch im äußeren verdampferseitigen Bauteil jeweils mindestens eine Öffnung eingelassen, die zum verdampferseitigen Kanalabschnitt gehört. Mindestens dann, wenn das Stellmittel in der Freigabe-Endposition ist, bevorzugt auch ab einer bestimmten Zwischen-Position, überlappen die oder eine Öffnung des inneren Bauteils und die oder eine Öffnung des äußeren Bauteils miteinander, und der verdampferseitige Kanalabschnitt ist durch diese beiden Öffnungen hindurch geführt und durchlässig.

In einer Ausgestaltung ist der Vorsprung des Kontaktprofils an dem Anschlussabschnitt, besonders bevorzugt an dem inneren verdampferseitigen Bauteil angeordnet. Beispielsweise fungiert der oben beschriebene Kopf als der Vorsprung. Die korrespondierende Aussparung ist an dem Adapter, besonders bevorzugt in dem behälterseitigen Kontaktprofil, angeordnet. Das äußere verdampferseitige Bauteil lässt sich dadurch relativ zu diesem Vorsprung bewegen.

Die Ausgestaltung, den Vorsprung des Kontaktprofils am Anschlussabschnitt und nicht am Adapter anzubringen, hat folgenden Vorteil: Der Narkosemittel-Verdampfer mit dem Anschlussabschnitt wird in der Regel in einem Anästhesiegerät verwendet und nur mitsamt des Anästhesiegerät bewegt.

Während der Nutzungszeit des Narkosemittel-Verdampfers wird in der Regel mehrmals erneut ein Narkosemittel-Behälter mit dem Narkosemittel-Verdampfers verbunden. Der Narkosemittel-Behälter mit dem Adapter wird hingegen zu diesem Anästhesiegerät transportiert. Ein Vorsprung im Adapter könnte unbeabsichtigt eingedrückt werden, ohne dass der Adapter mit dem Anschlussabschnitt verbunden ist, was dazu führt, dass der behälterseitige Kanalabschnitt geöffnet wird und Narkosemittel ausfließt oder anderweitig in die Umgebung entweicht, was unerwünscht ist.

In einer Ausgestaltung umgibt ein Gehäuse des Außenabschnitts wenigstens teilweise das äußere verdampferseitige Bauteil und damit auch das innere verdampferseitige Bauteil. Zwischen dem Gehäuse und dem äußeren verdampferseitigen Bauteil tritt ein Zwischenraum, bevorzugt ein Spalt auf. Falls der Adapter mit dem Anschlussabschnitt verbunden ist, so greift der Adapter in diesen Zwischenraum ein. Bevorzugt ist ein Teil des Adapters in diesen Spalt eingeführt.

In einer Fortbildung dieser Ausgestaltung liegt ein umlaufender Vorsprung des inneren verdampferseitigen Bauteils von einer Seite an dem Gehäuse an. Von der anderen Seite liegt der Adapter an dem Gehäuse an. Diese Ausgestaltung erhöht weiter die mechanische Stabilität und verringert weiter das Risiko, dass Narkosemittel austritt.

In einer Ausgestaltung ist der verdampferseitige Kanalabschnitt durch ein Element des inneren verdampferseitigen Bauteils hindurch geführt, beispielsweise durch eine Hülse hindurch. Ein Außenprofil dieses Elements, also beispielsweise dieser Hülse, zeigt zum Stellmittel hin. Wenn das Stellmittel in einer Endposition ist, liegt in einer Ausgestaltung dieses Außenprofil flächig an einem korrespondierenden Innenprofil des Stellmittels an. Zwischen dem Außenprofil und dem inneren Profil tritt bevorzugt kein Zwischenraum auf, in dem sich Narkosemittel ansammeln kann.

Weil die beiden Profile in der Sperr-Endposition aneinander anliegen, und zwar bevorzugt flächig, lässt sich diese gewünschte Wirkung des Stellmittels in der Sperr-Endposition ohne eine Dichtung erzielen. Eine solche Dichtung kann abnutzen oder von dem Narkosemittel angegriffen werden. In einer Ausgestaltung liegen die Profile dann flächig aneinander an, wenn das Stellmittel in der Freigabe-Endposition oder einer Zwischen-Position ist, also während eines Füllvorgangs. In einer Ausgestaltung liegen das Außenprofil des inneren verdampferseitigen Bauteils und das Innenprofil des Stellmittels dann flächig aneinander an, wenn das Stellmittel in der Freigabe-Endposition ist. Oder zwischen dem Außenprofil und dem Innenprofil tritt der kleinstmögliche Abstand auf. Wenn das Stellmittel in der Sperr-Endposition ist, tritt hingegen der maximal mögliche Abstand zwischen dem Innenprofil und dem Außenprofil auf. Diese Ausgestaltung erhöht die mechanische Stabilität und verringert weiter das Risiko, dass Narkosemittel austritt, wenn das Stellmittel in der Sperr-Endposition ist.

In einer Ausgestaltung wird ein Zwischenraum zwischen Außenprofil und Innenprofil gebildet. Dieser Zwischenraum steht in einer Fluidverbindung mit dem verdampferseitigen Kanalabschnitt.

Bevorzugt umfasst der Adapter ein äußeres behälterseitiges Bauteil und ein inneres behälterseitiges Bauteil. Das äußere behälterseitige Bauteil ist bevorzugt hohl und nimmt bevorzugt in seinem Inneren das innere behälterseitige Bauteil auf.

Bevorzugt ist der behälterseitige Kanalabschnitt zwischen dem inneren behälterseitigen Bauteil und dem äußeren behälterseitigen Bauteil hindurch geführt. Diese Ausgestaltung erspart die Notwendigkeit, in das innere behälterseitige Bauteil eine Öffnung anzubringen, durch welche der behälterseitige Kanalabschnitt hindurch geführt ist.

Erfindungsgemäß weist die Verbindungs-Anordnung einen Vorsprung und eine korrespondierende Aussparung auf. In einer Ausgestaltung weist das behälterseitige Kontaktprofil die Aussparung auf. Bevorzugt ist die Aussparung in dem inneren behälterseitigen Bauteil angeordnet.

Bevorzugt lässt sich das innere behälterseitige Bauteil relativ zum äußeren behälterseitigen Bauteil bewegen. Besonders bevorzugt umfasst der Adapter ein behälterseitiges Federelement. Dieses behälterseitige Federelement stützt sich bevorzugt am äußeren behälterseitigen Bauteil ab. Das behälterseitige Federelement ist bestrebt, das innere behälterseitige Bauteil auf den Anschlussabschnitt zu zu bewegen. Diese Ausgestaltung führt dazu, dass der behälterseitige Kanalabschnitt verschlossen oder anderweitig unterbrochen ist, solange das behälterseitige Federelement in der Ruheposition ist, und erst dann geöffnet wird, wenn das innere behälterseitige Bauteil relativ zum äußeren behälterseitigen Bauteil gegen die Kraft des behälterseitigen Federelements bewegt wird. Diese Ausgestaltung reduziert noch stärker die Gefahr, dass Narkosemittel ungewollt aus dem Narkosemittel-Behälter und durch den Adapter hindurch austritt.

In einer Fortbildung dieser Ausgestaltung umfasst das äußere behälterseitige Bauteil ein Abstützelement. Das behälterseitige Federelement stützt sich an diesem Abstützelement ab. Das Abstützelement zeigt zum Narkosemittel-Behälter hin und ist bevorzugt vom behälterseitigen Kontaktprofil weg gewölbt. Besonders bevorzugt ist das Abstützelement domförmig ausgestaltet.

Bevorzugt umfasst das äußere behälterseitige Bauteil weiterhin ein Anschlagelement. Dieses Anschlagelement ist bevorzugt mit dem Abstützelement mechanisch verbunden. Dieses Anschlagelement begrenzt eine Bewegung, welche das innere behälterseitige Bauteil auf den Narkosemittel-Behälter zu auszuführen vermag.

Bevorzugt weist dieses Abstützelement wenigstens eine Aussparung auf. Der behälterseitige Kanalabschnitt ist durch die oder durch mindestens eine Aussparung im Abstützelement hindurch geführt. Dadurch ist es nicht erforderlich, den behälterseitigen Kanalabschnitt um das Abstützelement herum zu führen. Diese Ausgestaltung erhöht in vielen Fällen die mechanische Stabilität verglichen mit anderen möglichen Ausgestaltungen des behälterseitigen Kanalabschnitts und / oder des Abstützelements.

Erfindungsgemäß umfasst der Anschlussabschnitt einen verdampferseitigen Kanalabschnitt. In einer Ausgestaltung umfasst dieser verdampferseitige Kanalabschnitt einen trichterförmigen Abschnitt. Dieser trichterförmige Abschnitt ist bevorzugt an demjenigen Ende des verdampferseitigen Kanalabschnitts angeordnet, welcher dem Narkosemittel-Verdampfer zugewandt ist. Der Durchmesser dieses trichterförmigen Abschnitts vergrößert sich in Richtung des Narkosemittel-Verdampfers. Entsprechend verjüngt der trichterförmigen Abschnitt sich in die entgegengesetzte Richtung.

Diese Ausgestaltung hat insbesondere den folgenden Vorteil: Wenn ein Narkosemittel-Tank des Narkosemittel-Verdampfers mit Narkosemittel gefüllt ist, so verdrängt das einströmende Narkosemittel aufgrund des höheren spezifischen Gewichts Luft oder ein anderes Gas aus dem Narkosemittel-Tank. Dieses Gas enthält oft einen Anteil an Narkosemittel. Daher soll dieses Gas nicht in die Umgebung entweichen, sondern in den Narkosemittel-Behälter gelangen. Häufig wird der Narkosemittel-Tank schräg von oben befüllt. Das verdrängte Gas steigt auf. Der trichterförmige Abschnitt sammelt das aufsteigende Gas und lenkt es in den Rest des verdampferseitigen Kanalabschnitts. Von dort gelangt das aufsteigende Gas in den verbundenen behälterseitigen Kanalabschnitt.

Die Ausgestaltung mit dem trichterförmigen Abschnitt erspart in vielen Fällen die Notwendigkeit, einen separaten Entlüftungskanal zum Entlüften des Narkosemittel-Tanks vorzusehen. Möglich ist aber auch, einen separaten Entlüftungskanal vorzusehen, wobei dieser Entlüftungskanal bevorzugt im trichterförmigen Abschnitt beginnt.

Erfindungsgemäß bilden die beiden Kontaktprofile einen Vorsprung und eine Aussparung aus. Bevorzugt sind sowohl die Aussparung als auch der Vorsprung rotationssymmetrisch. Besonders bevorzugt hat der Vorsprung die Form eines Kegelstumpfs oder eines Kegels mit abgerundetem Kopf. Die Aussparung hat die Form eines korrespondierenden Konus, der diesen Kegelstumpf oder Kegel aufnimmt. Die rotationssymmetrische Ausgestaltung erleichtert es, den Anschlussabschnitt mit dem Adapter zu verbinden. Die rotationssymmetrische Ausgestaltung erspart die Notwendigkeit, den Narkosemittel-Behälter zunächst in eine bestimmte Orientierung relativ zum Narkosemittel-Verdampfer zu bringen. Die Ausgestaltung des Vorsprungs als Kegelstumpf und die der Aussparung als korrespondierender Konus führen weiterhin dazu, dass der Adapter und damit der Narkosemittel-Behälter sicher gehalten werden und nicht abkippen können. Möglich, aber nicht erforderlich ist es, dass ein Benutzer den Narkosemittel-Behälter während des gesamten Befüllens hält. Besonders bevorzugt sind das innere verdampferseitige Bauteil und / oder das innere behälterseitige Bauteil ebenfalls rotationssymmetrisch.

Erfindungsgemäß lässt der Adapter sich lösbar mit dem Anschlussabschnitt verbinden, um eine fluiddichte Fluidverbindung zwischen den Narkosemittel-Behälter und dem Narkosemittel-Verdampfer herzustellen. Bevorzugt hält eine Halterung den Adapter und verhindert, dass der Narkosemittel-Behälter mit dem Adapter seitlich abkippt. Diese Ausgestaltung erspart in vielen Fällen die Notwendigkeit, dass ein Benutzer den Narkosemittel-Behälter dergestalt halten muss, dass der Narkosemittel-Behälter nicht abkippt, während Narkosemittel in einen Tank des Narkosemittel-Verdampfers fließt.

In einer Ausgestaltung dieser Halterung umfasst der Anschlussabschnitt eine Schnapphalterung. Der Adapter lässt sich durch eine lineare Bewegung in den Anschlussabschnitt einführen, bis die Schnapphalterung einrastet und den Adapter hält. Ein Benutzer merkt, wann die Schnapphalterung eingerastet ist. In einer anderen Ausgestaltung wird die Halterung durch eine Bajonettverbindung realisiert. Um eine Bajonettverbindung herzustellen, ist oft eine geringere Kraft erforderlich als beim Herstellen einer Verbindung durch eine Schnapphalterung, nämlich lediglich eine Drehbewegung bis zu einem Anschlag. Auch das Lösen einer Bajonettverbindung erfordert weniger Kraft. Die Drehbewegung des Anwenders wird in ein Drehmoment in eine vorgegebene Richtung umgewandelt. Bevorzugt umfasst diese Bajonettverbindung mindestens einen Bajonettvorsprung und mindestens eine korrespondierende Bajonettaussparung, in welche der oder ein Bajonettvorsprung eingreift. Möglich ist, dass der oder jeder Bajonettvorsprung am Anschlussabschnitt angeordnet ist und die oder jede Bajonettaussparung an dem Adapter. Möglich ist auch, dass der oder jeder Bajonettvorsprung an dem Adapter angeordnet ist und die oder jede Bajonettaussparung an dem Anschlussabschnitt. Auch eine Kombination dieser beiden Ausführungsformen ist möglich.

Bevorzugt sind der Anschlussabschnitt aus einem Metall und der Adapter aus einem Kunststoff gefertigt. Die Verwendung von Metall führt zu einem stabilen Anschlussabschnitt. Weil der Adapter aus Kunststoff ist, kann er reversibel elastisch nachgeben, wenn er mit dem Anschlussabschnitt verbunden ist, was die Gefahr eines unerwünschten Spalts und damit eines Totraums weiter verringert. Außerdem wird - verglichen mit einem Adapter aus Metall - ein Kontakt Metall - Metall verhindert, wobei ein solcher Kontakt oft unerwünscht ist.

In einer Ausgestaltung lässt sich der Adapter relativ zu dem Anschlussabschnitt in drei Positionen und somit drei Zustände verbringen, nämlich in einen beabstandeten Zustand, einen Koppel-Zustand und in einen Durchlass-Zustand. Im beabstandeten Zustand tritt ein Abstand zwischen den beiden Kontaktprofilen auf. Im Koppel-Zustand hält der Anschlussabschnitt den Adapter und verhindert, dass der Adapter relativ zum Anschlussabschnitt senkrecht oder schräg zu einer Verschieberichtung bewegt werden kann. Bevorzugt liegen im Koppel-Zustand die beiden Kontaktprofile - oder wenigstens jeweils ein Bereich beider Kontaktprofile - ohne Zwischenraum aneinander an. Mindestens ein Kanalabschnitt ist im Koppel-Zustand aber noch gesperrt, sodass kein Narkosemittel fließen kann. Aus dem Koppel-Zustand lässt sich der Adapter relativ zu dem Anschlussabschnitt in den Durchlass-Zustand bringen. Bei der Überführung in den Durchlass-Zustand sowie im Durchlass-Zustand liegen die beiden Kontaktprofile ohne Zwischenraum aneinander an. Im Durchlass-Zustand bilden die beiden dann geöffneten Kanalabschnitte den durchgehenden Kanal.

Diese Ausgestaltung ermöglicht es, in zwei Stufen die lösbare Fluidverbindung zwischen dem Adapter und dem Anschlussabschnitt und somit zwischen dem Narkosemittel-Behälter und dem Narkosemittel-Verdampfer herzustellen. Zunächst wird der Adapter in den Koppel-Zustand gebracht und in diesem gehalten. Im Koppel-Zustand kann noch kein Narkosemittel austreten. Daher ist möglich, die Position des Adapters zu korrigieren. Erst dann wird der Adapter in den Durchlass-Zustand verbracht, bevorzugt gegen die Kraft mindestens eines Federelements. In der Regel reicht eine rein lineare Bewegung aus, um den Adapter aus dem Koppel-Zustand in den Durchlass-Zustand zu überführen.

In einer Fortbildung wird die Ausgestaltung mit dem Koppel-Zustand kombiniert mit der Ausgestaltung, bei welcher der Anschlussabschnitt ein inneres und ein äußeres verdampferseitiges Bauteil umfasst und der Adapter ein inneres und ein äußeres behälterseitiges Bauteil umfasst. Im Koppel-Zustand berührt das äußere behälterseitige Bauteil das äußere verdampferseitige Bauteil, während zwischen dem inneren behälterseitigen Bauteil und dem inneren verdampferseitigen Bauteil noch ein Abstand auftritt.

Eine bevorzugte Fortbildung der Ausgestaltung mit dem Koppel-Zustand ist diese: In dem Koppel-Zustand sind beide Kanalabschnitte noch geschlossen, bevorzugt durch ein verdampferseitiges Federelement und ein behälterseitiges Federelement. Wird der Adapter auf den Anschlussabschnitt zu bewegt und wird dadurch die Verbindungs-Anordnung aus dem Koppel-Zustand in den Durchlass-Zustand überführt, so wird zunächst der verdampferseitige Kanalabschnitt geöffnet, bevorzugt gegen die Kraft des verdampferseitigen Federabschnitts. Der behälterseitige Kanalabschnitt bleibt noch geschlossen. Erst bei einer weiteren Bewegung des Adapters wird auch der behälterseitige Kanalabschnitt geöffnet, und ein durchgehender Kanal für Narkosemittel wird dann bereitgestellt. Falls später der Adapter wieder vom Anschlussabschnitt weg bewegt wird und dadurch die Verbindungs-Anordnung aus dem Durchlass-Zustand in den beabstandeten Zustand überführt wird, so wird zunächst der behälterseitige Kanalabschnitt geschlossen und erst dann der verdampferseitige Kanalabschnitt.

Dieses Merkmal hat folgenden Effekt, der insbesondere dann relevant ist, wenn nach dem Befüllen eines Narkosemittel-Tanks der Adapter wieder vom Anschlussabschnitt getrennt wird: Kein Narkosemittel kann aus dem Narkosemittel-Behälter in den Narkosemittel-Verdampfer fließen, sobald der behälterseitige Kanalabschnitt geschlossen ist. Narkosemittel, welches nach dem Schließen des behälterseitigen Kanalabschnitt im Adapter oder im Anschlussabschnitt verbleibt, kann jedoch noch durch den geöffneten verdampferseitigen Kanalabschnitt in den Narkosemittel-Verdampfer fließen, bis auch der verdampferseitige Kanalabschnitt geschlossen ist. Das Merkmal verringert somit weiter die Gefahr, dass Narkosemittel in die Umgebung austritt. Die Erfindung betrifft weiterhin einen Anschlussabschnitt für einen Narkosemittel-Verdampfer, der sich mit einem korrespondierenden Adapter eines Narkosemittel-Behälters lösbar verbinden lässt. Weiterhin betrifft die Erfindung einen Adapter für einen Narkosemittel-Behälter, der sich mit einem korrespondierenden Anschlussabschnitt eines Narkosemittel-Verdampfers verbinden lässt. Die vorteilhaften Ausgestaltungen des Anschlussabschnitts und die des Adapters der Verbindungs-Anordnung sind auch vorteilhafte Ausgestaltungen dieses Anschlussabschnitts bzw. dieses Adapters.

Ein Narkosemittel-Verdampfer mit einem erfindungsgemäßen Anschlussabschnitt umfasst in einer Ausgestaltung einen eigenen Narkosemittel-Tank. Der verdampferseitige Kanalabschnitt steht in Fluidverbindung mit dem Narkosemittel-Tank. Dieser Tank lässt sich mit Narkosemittel aus einem Narkosemittel-Behälter auffüllen, wobei dieser Narkosemittel-Behälter einen Adapter umfasst und der Adapter und der Anschlussabschnitt zusammen eine erfindungsgemäße Verbindungs-Anordnung bilden. Diese Ausgestaltung vermeidet die Notwendigkeit, dass beim Einsatz des Narkosemittel-Verdampfers, also beim Verdampfen von Narkosemittel, der Narkosemittel-Behälter mit dem Narkosemittel-Verdampfer verbunden ist. In einer anderen Ausgestaltung fungiert der Narkosemittel-Behälter selber als der Narkosemittel-Tank, der beim Einsatz des Narkosemittel-Verdampfers verwendet wird. Diese Ausgestaltung erspart die Notwendigkeit, einen eigenen Narkosemittel-Tank für den Narkosemittel-Behälter vorsehen zu müssen. Der Narkosemittel-Verdampfer kann kleiner ausgestaltet sein.

Im Folgenden wird die Erfindung anhand eines Ausführungsbeispiels beschrieben.

Hierbei zeigen:
- Figur 1: das innere verdampferseitige Bauteil gemäß einer ersten Ausführungsform des Anschlussabschnitts des Narkosemittel-Verdampfers in einer Querschnittsdarstellung;
- Figur 2: das äußere verdampferseitige Bauteil gemäß der ersten Ausführungsform des Anschlussabschnitts in einer Querschnittsdarstellung;
- Figur 3: die erste Ausführungsform des Anschlussabschnitts in einer Blickrichtung von oben und parallel zur Mittelachse;
- Figur 4: das äußere verdampferseitige Bauteil der ersten Ausführungsform des Anschlussabschnitts in der Blickrichtung von Figur 3;
- Figur 5: die erste Ausführungsform des Anschlussabschnitts in einer Blickrichtung von unten;
- Figur 6: das innere verdampferseitige Bauteil gemäß einer zweiten Ausführungsform in einer Querschnittsdarstellung;
- Figur 7: den gesamten Anschlussabschnitt gemäß der zweiten Ausführungsform in einer Querschnittsdarstellung;
- Figur 8: den Adapter des Narkosemittel-Behälters gemäß einer ersten Ausführungsform in einer Querschnittsdarstellung;
- Figur 9: den Adapter des Narkosemittel-Behälters gemäß einer zweiten Ausführungsform in einer Querschnittsdarstellung;
- Figur 10: eine erste Situation beim Herstellen des verdampferseitigen Bauteils: Dichtring noch nicht eingesetzt;
- Figur 11: eine zweite Situation beim Herstellen des verdampferseitigen Bauteils: Dichtring eingesetzt, inneres und äußeres verdampferseitiges Bauteil vor dem Zusammensetzen;
- Figur 12: eine dritte Situation beim Herstellen des verdampferseitigen Bauteils: Kopf wird auf die Hülse des inneren verdampferseitigen Bauteils aufgesetzt;
- Figur 13: eine vierte Situation beim Herstellen des verdampferseitigen Bauteils: Bauteil zusammengesetzt;
- Figur 14: die komplette Verbindungs-Anordnung mit dem Anschlussabschnitt und dem Adapter;
- Figur 15: die komplette Verbindungs-Anordnung in einem Koppel-Zustand in einer Seitenansicht;
- Figur 16: die Verbindungs-Anordnung von Figur 15 in einer Draufsicht senkrecht von oben;
- Figur 17: in einer Übersichtsdarstellung eine Bajonettverbindung zwischen Anschlussabschnitt und Adapter;
- Figur 18: Details der Bajonettverbindung von Figur 17.

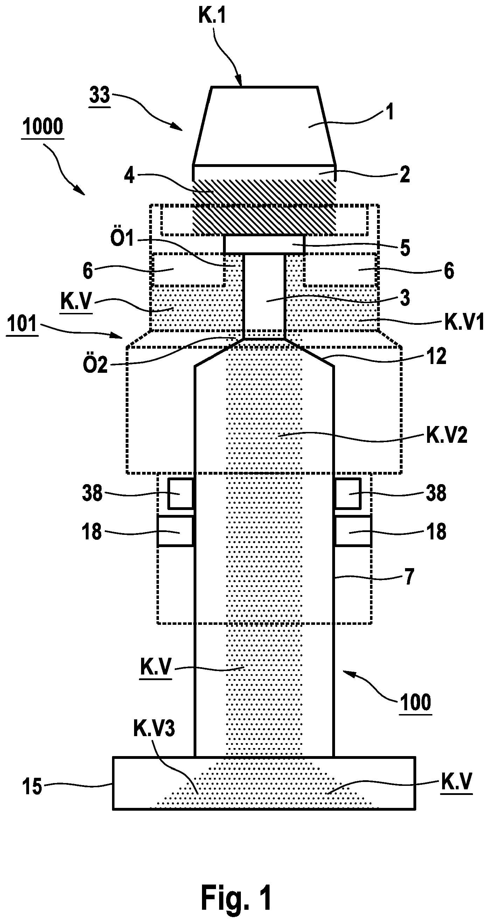

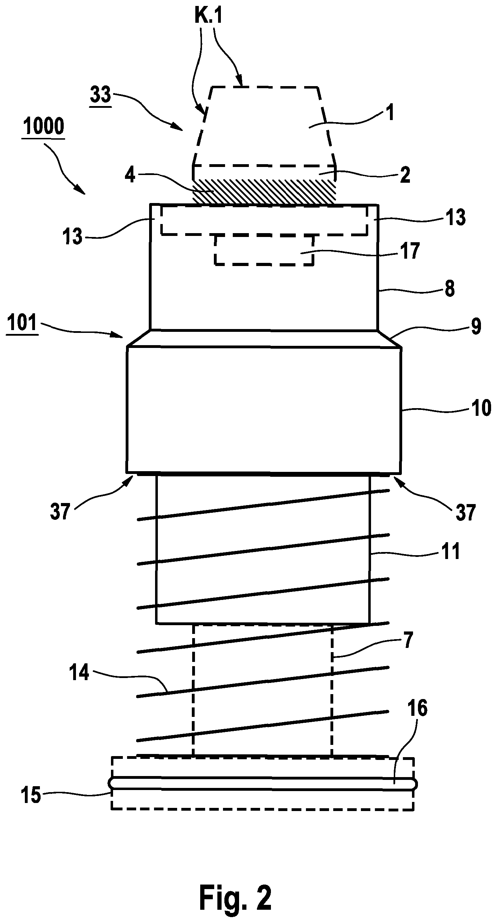

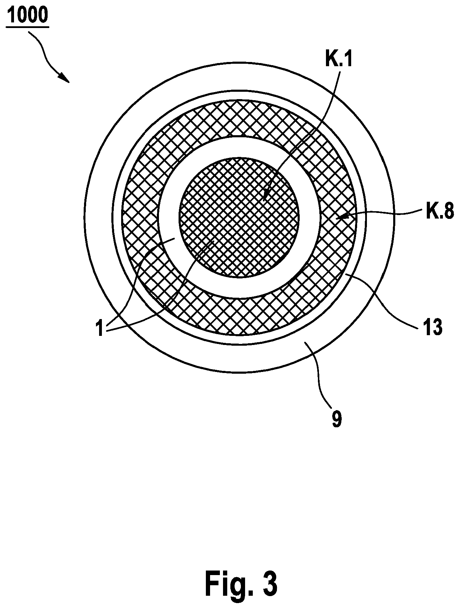

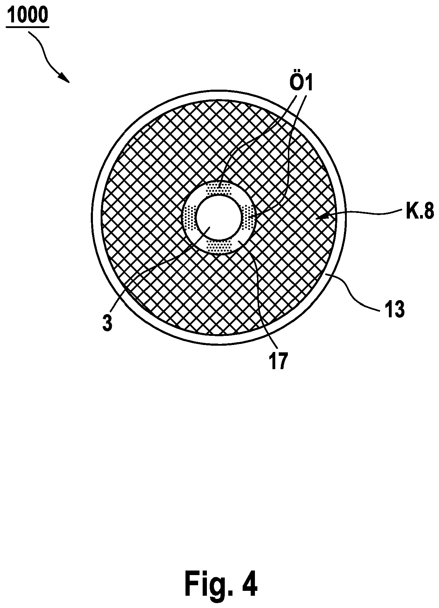

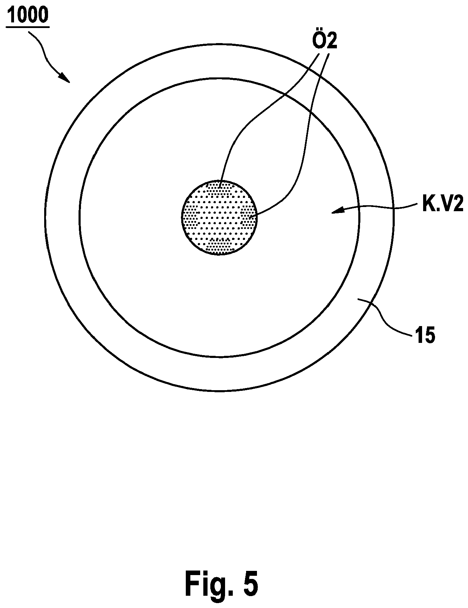

Die Verbindungsanordnung des Ausführungsbeispiels umfasst einen Anschlussabschnitt 1000, der in einen Narkosemittel-Verdampfer 300 eingesetzt ist, und einen Adapter 1100, der in einen Narkosemittel-Behälter 400 eingesetzt ist. Der Anschlussabschnitt 1000 des Ausführungsbeispiels umfasst ein inneres verdampferseitiges Bauteil 100 und ein äußeres verdampferseitiges Bauteil 101. Figur 1 bis Figur 5 zeigen eine erste Ausführungsform des Anschlussabschnitts 1000 des Narkosemittel-Verdampfers 300. Figur 1 und Figur 2 zeigen das innere verdampferseitige Bauteil 100 bzw. das äußere verdampferseitige Bauteil 101 in einer Querschnittsdarstellung durch die Mittelachse des rotationssymmetrischen Anschlussabschnitts 1000. In Figur 1 ist das äußere verdampferseitige Bauteil 101 mit gepunkteten Linien dargestellt. In Figur 2 ist das innere verdampferseitige Bauteil 100 mit gestrichelten Linien dargestellt. Figur 3 und Figur 4 zeigen den Anschlussabschnitt 1000 in einer Blickrichtung von oben und parallel zur Mittelachse der Bauteile 100 und 101, wobei in Figur 4 das innere verdampferseitige Bauteil 100 fortgelassen ist. Figur 5 zeigt den Anschlussabschnitt 1000 in einer Blickrichtung von unten, welche der Blickrichtung von Figur 3 und Figur 4 entgegengesetzt ist.

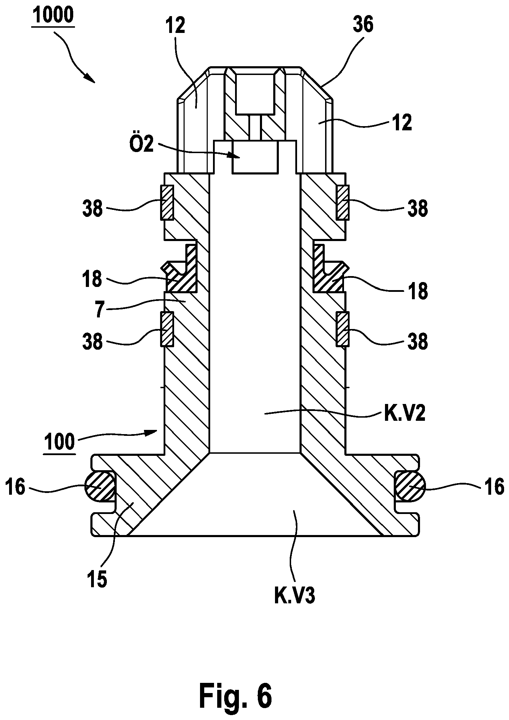

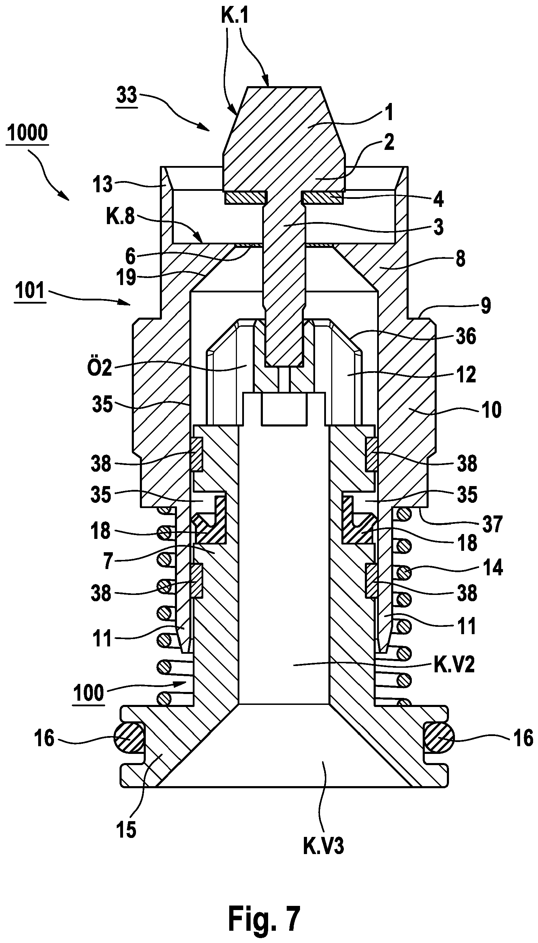

Figur 6 und Figur 7 zeigen in einer Querschnittsdarstellung eine zweite Ausführungsform des Anschlussabschnitts 1000. In Figur 6 ist das innere verdampferseitige Bauteil 100 dargestellt, in Figur 7 der gesamte Anschlussabschnitt 1000 der zweiten Ausführungsform.

Das innere verdampferseitige Bauteil 100 umfasst in beiden Ausführungsformen (in einer Reihenfolge von oben nach unten)
- einen Kopf 33 mit einem Kegelstumpf 1 und einer angrenzenden Scheibe 2, wobei der Kegelstumpf 1 fest mit der Scheibe 2 verbunden ist,
- einen umlaufenden Dichtring 4, der an der Scheibe 2 montiert ist,
- einen Hals 3, der bevorzugt innen hohl ist,
- eine Scheibe 5 um den Hals 3, die an den Dichtring 4 angrenzt,
- einen Kragen 12, der fest mit dem Hals 3 verbunden ist und den Hals 3 aufnimmt,
- eine innen hohle Röhre 7, die fest mit dem Kragen 12 verbunden ist,
- eine Dichtscheibe 18 an der Außenwand der Röhre 7,
- optional mehrere Gleitringe 38 an der Außenwand der Röhre 7 (vgl. Figur 7) und
- eine überstehende Scheibe 15 am Fuß des Bauteils 100, die fest mit der Röhre 7 verbunden ist.

Die überstehende Scheibe 15 umfasst eine umlaufende Rinne, in welche ein Dichtring 16 aufgenommen ist. Bevorzugt sind alle diese Elemente rotationssymmetrisch ausgestaltet und koaxial zu einer Mittelachse des inneren verdampferseitigen Bauteils 100 angeordnet.

Der pilzförmige Kopf 33 hat einen maximalen Außendurchmesser, welcher größer als derjenige des Halses 3 und derjenige der Scheibe 5 ist. In einer Ausgestaltung wird der gesamte Kopf 33 und optional zusätzlich der Hals 3 aus einem Werkstück hergestellt, beispielsweise durch Zerspanen.

Im Ausführungsbeispiel sind der Dichtring 4, der Dichtring 16 und die Dichtscheibe 18 aus einem elastischen Material gefertigt. Alle anderen Elemente des inneren verdampferseitigen Bauteils 100 sind aus einem festen Material, bevorzugt aus einem Metall, gefertigt.

Das äußere verdampferseitige Bauteil 101 umfasst (in einer Reihenfolge von oben nach unten)
- ein Stellmittel 8 in Form einer Hülse,
- ein umlaufender Vorsprung 13 oben am Stellmittel 8, wobei der umlaufenden Vorsprung 13 eine ebene Fläche in Form eines Kreisrings umschließt,
- einen Kragen 9, der fest mit dem Stellmittel 8 verbunden ist,
- eine größere Hülse 10, die fest mit dem Kragen 9 verbunden ist, d.h. der Kragen 9 verbindet das Stellmittel 8 fest mit der größeren Hülse 10,
- eine kleinere Hülse 11, die fest mit der größeren Hülse 10 verbunden ist, und
- eine kreisringförmige oder angeschrägte Übergangsfläche 37 zwischen den Hülsen 10 und 11.

In einer Ausgestaltung werden das Stellmittel 8, die größere Hülse 10 und die kleinere Hülse 11 aus einem einzigen Bauteil hergestellt, beispielsweise durch Zerspanen.

Der Anschlussabschnitt 1000 umfasst weiterhin ein verdampferseitiges Federelement 14 in Form einer spiralförmigen Druckfeder, welches die kleinere Hülse 11 umgibt, an der Übergangsfläche 37 unten an der größeren Hülse 10 anliegt, sich an der überstehenden Scheibe 15 abstützt und die Hülse 7 umgibt. Das obere Ende des Stellmittels 8 bildet eine ebene Kontaktfläche K.8 in Form eines Kreisrings, der senkrecht auf der Mittelachse des äußeren verdampferseitigen Bauteils 101 steht. Im Inneren dieser Kontaktfläche K.8 ist eine kreisringförmige Aussparung 17 eingelassen. Der Hals 3 ist durch diese Aussparung 17 hindurch geführt. Die Innenwand des Stellmittels 8 umfasst ein konusförmiges Segment 19. Das äußere verdampferseitige Bauteil 101 umfasst weiterhin eine zylinderförmige Innenwand 35, welche vom Stellmittel 8 und von den beiden Hülsen 10 und 11 gebildet wird, vgl. Figur 7). Bevorzugt sind alle diese Elemente rotationssymmetrisch und koaxial zu einer Mittelachse des äußeren verdampferseitigen Bauteils 101 angeordnet. Die Mittelachsen der beiden Bauteile 100 und 101 stimmen vorzugsweise überein. Dank der Gleitringe 38 kann das äußere verdampferseitige Bauteil 101 sich relativ zum inneren verdampferseitigen Bauteil 100 um die gemeinsame Mittelachse drehen.

Bevorzugt ist das gesamte äußere verdampferseitige Bauteil 101 aus einem Metall hergestellt.

Das äußere verdampferseitige Bauteil 101 lässt sich linear entlang der gemeinsamen Mittelachse relativ zu dem inneren verdampferseitigen Bauteil 100 bewegen. Die verdampferseitige Druckfeder 14 ist bestrebt, das äußere verdampferseitige Bauteil 101 von der Scheibe 15 (dem Fuß) weg zu bewegen. Diese Bewegung wird in einer Ausgestaltung durch den umlaufenden Dichtring 4 begrenzt. In einer Endposition, in der die Druckfeder 14 die größtmögliche Länge erreicht hat, grenzt die Kontaktfläche K.8 an diesen Dichtring 4 an. In dieser Position greift die Scheibe 5 in die Aussparung 17 ein.

In einer Ausgestaltung wird die Bewegung des äußeren verdampferseitigen Bauteils 101 in die entgegengesetzte Richtung dadurch begrenzt, dass das Innenprofil 19 des äußeren verdampferseitigen Bauteils 101 das Außenprofil 36 des inneren verdampferseitigen Bauteils 100 berührt. In einer anderen Ausgestaltung kann die kleinere Hülse 11 die Scheibe 15 erreichen, wenn die Druckfeder 14 entsprechend weit zusammengedrückt wird, und die Scheibe 15 definiert eine Endposition.

Im Inneren des Anschlussabschnitts 1000 wird ein verdampferseitiger Kanalabschnitt K.V ausgebildet, der in Figur 1 als punktierte Fläche dargestellt ist. Dieser verdampferseitige Kanalabschnitt K.V umfasst
- ein Segment K.V1, das im Inneren des Stellmittels 8 angeordnet ist,
- ein Segment K.V2, das durch die Röhre 7 hindurch geführt ist, und
- ein trichterförmiges Segment K.V3, das im Inneren der Scheibe 15 angeordnet ist, wobei der Durchmesser dieses Trichters sich in Richtung auf die Röhre 7 und somit auf das Segment K.V2 zu verkleinert.

Das Segment K.V1 steht in Fluidverbindung mit mehreren Öffnungen Ö1 in der Kontaktfläche K.8. Diese Öffnungen Ö1 grenzen an den Hals 3 an. In der in Figur 1 gezeigten Endposition verschließen der Dichtring 4 und optional die Scheibe 2 diese Öffnungen Ö1. Diese Position ist daher eine Sperr-Endposition des Stellmittels 8. Das Stellmittel 8 liegt dann formschlüssig an dem Dichtring 4 an. Die andere Endposition wird erreicht, wenn die Druckfeder 14 so weit wie möglich zusammengedrückt ist und in der in Figur 7 gezeigten Ausgestaltung die kleinere Hülse 11 an der Scheibe (dem Fuß) 15 anliegt. Diese Endposition ist die Freigabe-Endposition des Stellmittels 8. Figur 1 und Figur 2 zeigen das Stellmittel 8 in der Sperr-Endposition. In der Freigabe-Endposition liegt die konusförmige Innenwand 19 flächig an der Außenwand 36 des Kragens 12 an oder hat - so die Ausgestaltung gemäß Figur 7 - den kleinstmöglichen Abstand zu der Außenwand 36. Bevorzugt haben die Innenwand 19 und die Außenwand 36 das gleiche Profil. Figur 7 zeigt eine Zwischen-Position zwischen der Freigabe-Endposition und der Sperr-Endposition, in welcher der verdampferseitige Kanalabschnitt K.V bereits geöffnet ist.

Zwischen der Innenwand 19 und der Außenwand 36 kann ein Zwischenraum auftreten. Dieser Zwischenraum steht aber ständig in Fluidverbindung mit dem Narkosemittel-Tank 304, so dass sich kein Narkosemittel in diesem Zwischenraum sammeln kann.

An dem zum Kopf 33 hin zeigenden Ende ist die Röhre 7 des inneren verdampferseitigen Bauteils 100 zum Hals 3 hin gewölbt, wodurch der Kragen 12 ausgebildet wird, vgl. Figur 1. In dieses gewölbte Ende 12 sind mehrere Öffnungen Ö2 eingelassen, vgl. Figur 5. Diese Öffnungen Ö2 verbinden dann, wenn das Stellmittel 8 in der Freigabe-Endposition oder einer Zwischen-Position ist, zusammen mit den Öffnungen Ö1 das Segment K.V2 mit dem Segment K.V1. Bevorzugt überlappen die Öffnungen Ö2 - gesehen in eine Richtung parallel zur Mittelachse der Bauteile 100 und 101 - wenigstens teilweise mit den Öffnungen Ö1. Das trichterförmige Segment K.V3 steht dauerhaft in Fluidverbindung mit dem Segment K.V2.

Der Narkosemittel-Verdampfer 300 hält das Narkosemittel auf einer Temperatur von z.B. 40° C. Ein Narkosemittel-Tank 304 steht oft unter Überdruck. Dieser Überdruck pflanzt sich in die Segmente K.V2 und K.V3 fort und wirkt in die gleiche Richtung wie die Druckfeder 14. Die Druckfeder 14 hat bevorzugt eine so große Federkraft, und die Öffnungen Ö1, Ö2 sind so klein, dass die Druckfeder 14 - unterstützt durch den Überdruck -das Stellmittel 8 rasch in die Sperr-Endposition schiebt, so dass nur wenig Narkosemittel aus dem Narkosemittel-Verdampfer 300 entweichen kann.

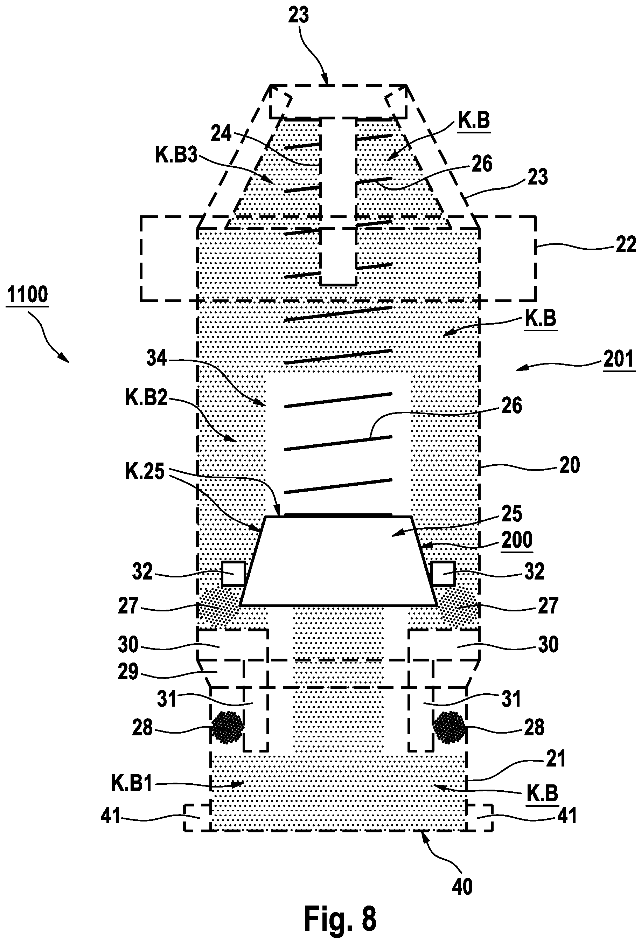

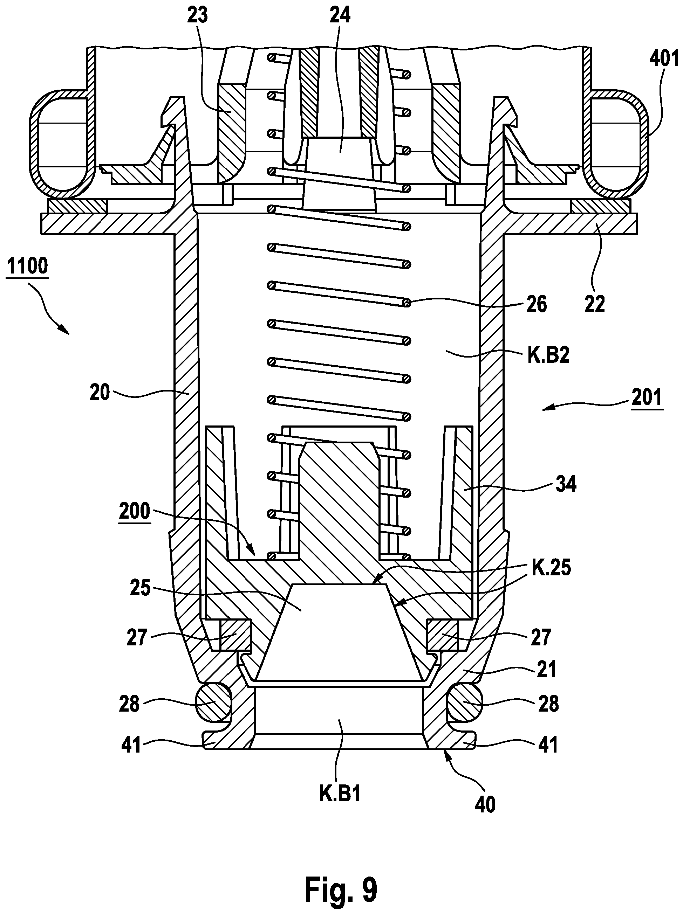

Figur 8 und Figur 9 zeigen zwei leicht unterschiedliche Ausführungsformen des Adapters 1100. Dieser Adapter 1100 ist in den Narkosemittel-Behälter 400 eingesetzt, und zwar bevorzugt lösbar, und umfasst ein inneres behälterseitiges Bauteil 200 und ein äußeres behälterseitiges Bauteil 201. Das innere behälterseitige Bauteil 200 ist in Figur 8 mit durchgezogenen Linien dargestellt, das äußere behälterseitige Bauteil 201 mit gestrichelten Linien.

Das innere behälterseitige Bauteil 200 umfasst
- eine trichterförmige Hülse 25,
- eine Röhre oder Hülse 34, die mit der trichterförmigen Hülse 25 fest verbunden ist und in einer Ausgestaltung (Figur 9) Öffnungen aufweist,
- ein Dichtring 27, der außen an der Hülse 34 (Figur 9) oder an der Hülse 25 (Figur 8) befestigt ist, und
- optional eine Halterung 32 für den Dichtring 27, die an der trichterförmigen Hülse 25 befestigt ist.

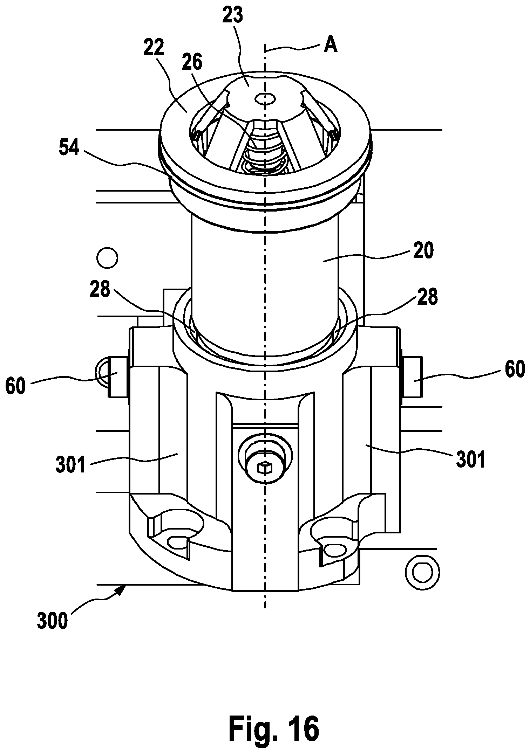

Das äußere behälterseitige Bauteil 201 umfasst (von oben nach unten)
- ein Abstützelement 23, welches bevorzugt domförmig oder zylindrisch ausgestaltet ist, also sich in den Narkosemittel-Behälter 300 hinein wölbt und mehrere Aussparungen aufweist,
- eine Röhre 24, die an dem Abstützelement 23 befestigt ist,
- eine Dichtscheibe 22, die in einer korrespondierenden Öffnung in einer Wand 401 des Narkosemittel-Behälters 400 aufgenommen ist,
- einen Dichtring 54, der in einer Rinne der Dichtscheibe 22 aufgenommen ist (vgl. Figur 16),
- eine größere Hülse 20,
- eine umlaufende Scheibe 30, die fest im Inneren der größeren Hülse 20 angeordnet ist,
- einen Kragen 29, der mit der größeren Hülse 20 fest verbunden ist,
- eine kleinere Hülse 21, die mit dem Kragen 29 fest verbunden ist, d.h. der Kragen 29 verbindet die beiden Hülsen 20 und 21 fest miteinander,
- eine umlaufende Halterung 31, die fest mit der umlaufenden Scheibe 30 verbunden ist, und
- ein Dichtring 28, der in einer Ausgestaltung (Figur 8) den Raum zwischen der Halterung 31 und der Innenwand der kleineren Hülse 21 abdeckt und in einer anderen Ausgestaltung (Figur 9) außen an der größeren Hülse 20 angeordnet ist.

Bevorzugt sind sowohl das äußere behälterseitige Bauteil 200 als auch das innere behälterseitige Bauteil 201 aus Kunststoff hergestellt - bis auf die Dichtringe 27 und 28. Diese Ausgestaltung spart Gewicht ein. Der Adapter 1100 aus Kunststoff lässt sich reversibel verformen.

Der Adapter 1100 umfasst weiterhin ein behälterseitiges Federelement 26, das sich am Abstützelement 23 abstützt, an der trichterförmigen Hülse 25 anliegt und die Form einer Druckfeder hat.

Im Inneren des Adapters 1100 ist ein behälterseitiger Kanalabschnitt K.B ausgebildet, der als punktierte Fläche dargestellt ist und folgende Segmente umfasst:
- ein Segment K.B1, das im Inneren der kleineren Hülse 21 angeordnet ist,
- ein Segment K.B2, das im Inneren der größeren Hülse 20 angeordnet ist, und
- ein Segment K.B3, das im Inneren des Abstützelements 23 angeordnet ist.

Die behälterseitige Druckfeder 26 ist bestrebt, das innere behälterseitige Bauteil 200 relativ zum äußeren behälterseitigen Bauteil 201 in eine Endposition zu drücken. In dieser Endposition trennt die trichterförmigen Hülse 25 das Segment K.B1 von den Segment K.B2, so dass diese Endposition eine Sperr-Endposition ist, in welcher die Halterung 32 und der Dichtring 27 an der Scheibe 30 anliegen. Wenn eine Kraft von unten auf die trichterförmige Hülse 25 ausgeübt wird, also durch die Hülse 21 hindurch, so wird die Druckfeder 26 zusammengedrückt, die Hülse 25 wird von der Scheibe 30 weggedrückt, und eine Fluidverbindung zwischen den Segment K.B1 und K.B2 wird erzeugt. Die Segmente K.B2 und K.B3 stehen dauerhaft in Fluidverbindung miteinander.

Die trichterförmige Hülse 25 ist vollständig im Inneren des äußeren behälterseitigen Bauteils 201 angeordnet und hat in Richtung der Mittelachse einen Abstand zum unteren Ende 40 dieses Bauteils 201. Dadurch wird die Gefahr reduziert, dass die Hülse 25 unbeabsichtigt gedrückt wird, die Segmente K.B1 und K.B2 miteinander verbunden werden und das innere behälterseitige Bauteil 200 dadurch aus der Sperr-Endposition in Richtung der Freigabe-Endposition bewegt wird und Narkosemittel entweichen kann.

In der zweiten Ausführungsform des Adapters 1100, die in Figur 9 gezeigt wird, ist eine umlaufende Kontaktfläche 40 in flächigem Kontakt mit der Kontaktfläche K.8 des Stellmittels 8. In der ersten Ausführungsform gemäß Figur 8 liegt die Kontaktfläche K.8 hingegen an dem Dichtring 28 mit der Hülse 31 an. Außerdem ist ein umlaufender Vorsprung 41 dargestellt. In beiden Ausgestaltungen wird folgendes bewirkt: Wenn der Adapter 1100 auf den Anschlussabschnitt 1000 aufgesetzt wird, kommen die behälterseitigen Kontaktflächen 28 und 31 bzw. 40 in Kontakt mit der Kontaktfläche K.8. Außerdem kommt die Kontaktfläche K.1 in Kontakt mit der Kontaktfläche K.25.

Wenn der Adapter 1100 weiter auf den Fuß 15 zu verschoben wird, drückt die behälterseitige Kontaktfläche 28 und 31 bzw. 40 die anliegende Kontaktfläche K.8 und damit das Stellmittel 8 gegen die Kraft der Druckfeder 14 aus der Sperr-Endposition in die Freigabe-Endposition.

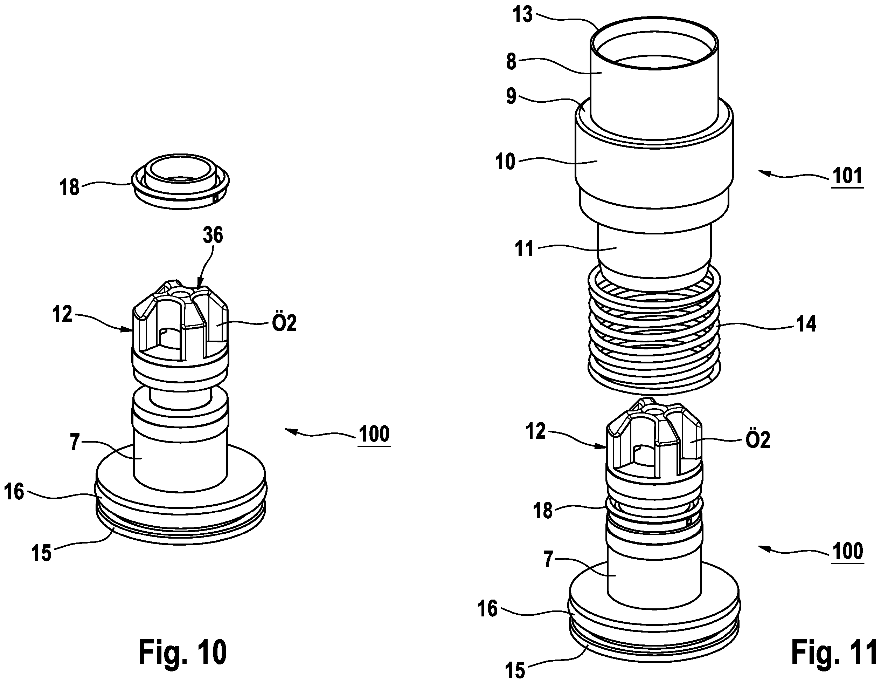

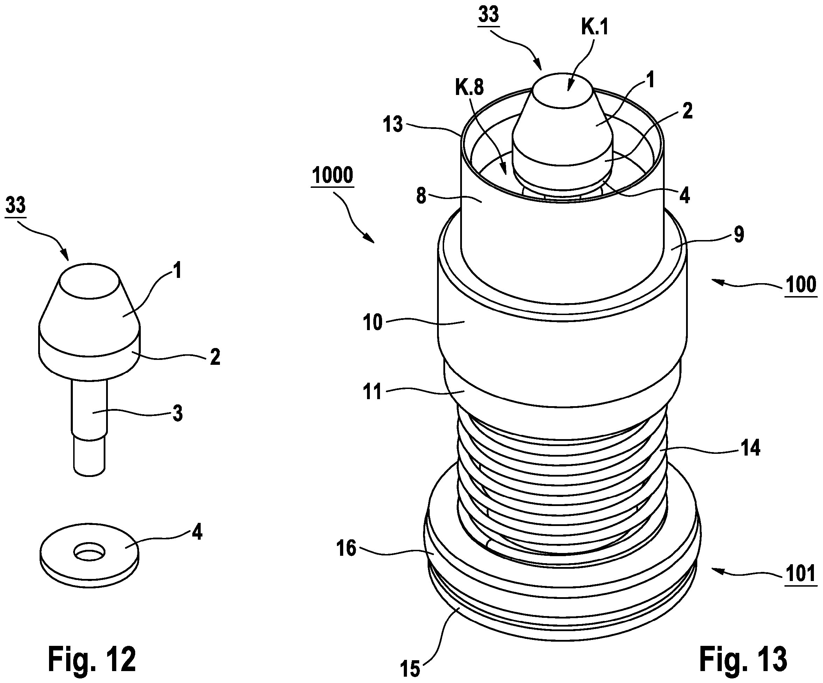

Figur 10 bis Figur 13 zeigen in einer perspektivischen Darstellung eine Ausgestaltung, wie der Anschlussabschnitt 1000 zusammengesetzt wird. Die Dichtscheibe 18 wird in eine passende Aussparung in der Röhre 7 eingesetzt. Außerdem wird der Dichtring 16 in die kreisförmige Rinne im Fuß 15 gesetzt. Figur 10 zeigt das innere verdampferseitige Bauteil 100 in einer perspektivischen Darstellung vor dem Einsetzen der Dichtscheibe 18, Figur 11 in einer perspektivischen Darstellung nach dem Einsetzen. Das Stellmittel 8 mit dem umlaufenden Vorsprung 13, der Kragen 9, die größere Hülse 10 und die kleinere Hülse 11 werden fest miteinander verbunden. Anschließend werden die behälterseitige Druckfeder 14 und die verbundenen Teile 8, 9, 10, 11 von oben über die Röhre 7 gestülpt, vgl. Figur 11. Die Dichtscheibe 4 wird von unten über den Hals 3 und die Scheibe 2 gestülpt, vgl. Figur 12. Die Teile werden zusammengesetzt, vgl. Figur 13. Die beiden verdampferseitigen Bauteile 100 und 101 lassen sich im regulären Betrieb nicht voneinander trennen, sondern werden linear verschiebbar in einer koaxialen Position gehalten.

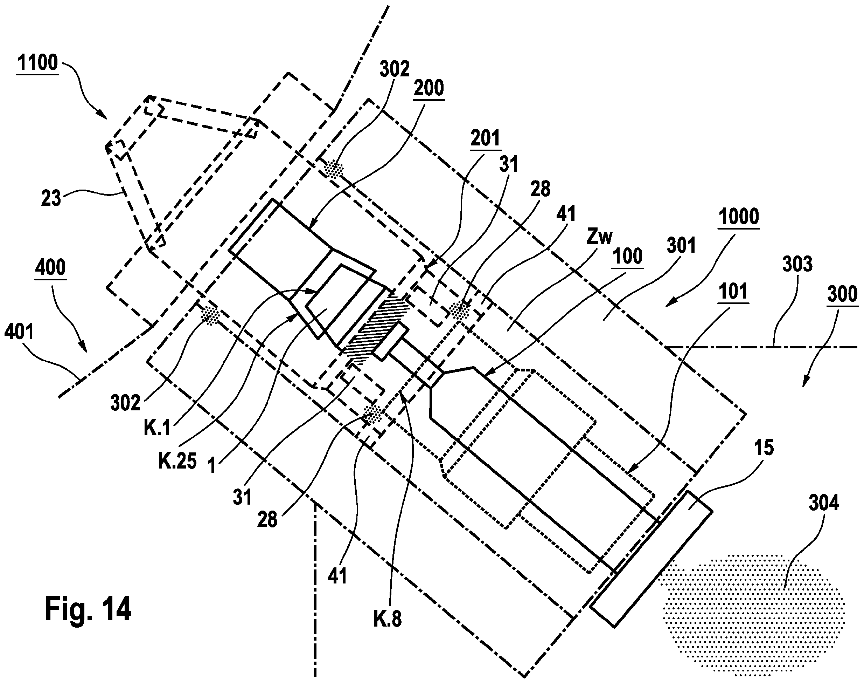

Figur 14, Figur 15 und Figur 16 zeigen in einer Seitenansicht bzw. in einer Draufsicht, wie ein Narkosemittel-Behälter 400 mit einem erfindungsgemäßen Adapter 1000 schräg auf einen Narkosemittel-Verdampfer 300 mit einem erfindungsgemäßen Anschlussabschnitt 1000 aufgesetzt ist. Bevorzugt sind sowohl der Adapter 1100 als auch der Anschlussabschnitt 1000 rotationssymmetrisch um die Achse A, so dass es nicht erforderlich ist, den Adapter 1100 in eine bestimmte Orientierung relativ zum Anschlussabschnitt 1000 zu bringen.

Zum Anschlussabschnitt 1000 gehört ein schematisch gezeigtes Gehäuse 301, welches eine zylinderförmigen Röhre aufweist, die das äußere verdampferseitige Bauteil 101 umgibt. Das äußere verdampferseitige Bauteil 101 wird in der gezeigten Ausgestaltung mithilfe von mehreren Schrauben 60 in dem Gehäuse 301 gehalten. Diese Schrauben umfassend bevorzugt federnd gelagerte kugelförmige Druckstücke, welche das Bauteil 101 berühren. Möglich ist auch, dass das Bauteil 101 mithilfe von Rollen an den Schrauben 60 oder ohne Schrauben mithilfe von Federelementen gehalten wird, wobei die Federelemente optional eine Schnapphalterung bilden.

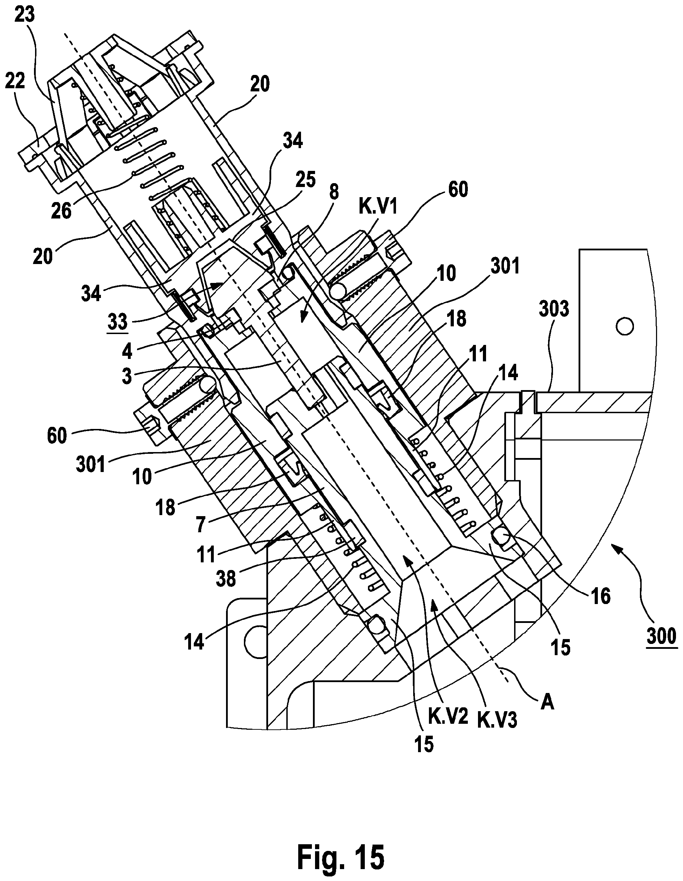

Beim Aufsetzen wird der Adapter 1100 zunächst in einen Koppel-Zustand relativ zum Anschlussabschnitt 1000 verbracht. Figur 15 zeigt diesen Koppel-Zustand. Die größere Hülse 20 des Adapters 1100 greift nunmehr in den Zwischenraum Zw zwischen der zylinderförmigen Innenwand des Gehäuses 301 und der Außenkontur des äußeren verdampferseitigen Bauteils 101 ein, umgibt das Stellmittel 8 des Anschlussabschnitts 1000 und liegt auf dem Kragen 9 auf. Der umlaufende Vorsprung 41 liegt flächig an der Innenwand des Gehäuses 301 an. In diesem Koppel-Zustand berührt das äußere behälterseitige Bauteil 201 das äußere verdampferseitige Bauteil 101, während zwischen dem inneren behälterseitigen Bauteil 200 und dem inneren verdampferseitigen Bauteil 100 noch ein Abstand in der Verschieberichtung auftritt. Im Koppel-Zustand berühren sich gegenüberliegende Kontaktfläche des Adapters 1100 und des Anschlussabschnitts 1000. Der Dichtring 28 und die Halterung 31 des äußeren behälterseitigen Bauteils 201 gemäß der ersten Ausführungsform nach Figur 8 liegen an der Kontaktfläche K.8 des äußeren verdampferseitigen Bauteils 101 an. Der umlaufende Vorsprung 13 drückt den Dichtring 28 zusammen. In der zweiten Ausführungsform nach Figur 9 liegt der Dichtring 27 an der Kontaktfläche K.8 an. In Figur 14 berühren sich einerseits die Kontaktfläche K.25 der trichterförmigen Hülse 25 und die Kontaktfläche K.1 des Kegelstumpfs 1 und andererseits der Dichtring 28 im Inneren der Hülse 21 und die Kontaktfläche K.8 des Stellmittels 8. Der Kontakt zwischen den Kontaktfläche K.25 und K.1 verhindert, dass der Adapter 1100 seitlich bewegt wird. Die trichterförmige Form der Hülse 25 und der Kegelstumpf 1 führen zusammen den Adapter 1100 in eine zentrierte Position relativ zum Anschlussabschnitt 1000.

In diesem Koppel-Zustand sind die trichterförmige Hülse 25 und das Stellmittel 8 noch in einem Sperr-Zustand, und Narkosemittel kann noch nicht fließen. In Figur 15 ist ein Abstand zwischen der Hülse 15 und dem Kopf 33 mit dem Kegelstumpf 1 zu sehen. Im Koppel-Zustand halten die beiden noch entspannten Druckfedern 14 und 26 diese Sperrzustände aufrecht.

Nunmehr wird der Adapter 1100 linear auf den Narkosemittel-Verdampfer 300 zu bewegt. Je nach Ausgestaltung drückt der Dichtring 28 auf die Kontaktfläche K.8 des Stellmittels 8 oder die Hülse 20 auf den Kragen 9. Dadurch werden das Stellmittel 8 und damit das gesamte äußere verdampferseitige Bauteil 101 auf den Fuß 15 zu verschoben. Das Stellmittel 8 wird in die Freigabe-Endposition verschoben und gibt die Öffnungen Ö2 frei. Der Dichtring 4 deckt nicht mehr die Öffnungen Ö1 ab, sodass der verdampferseitige Kanalabschnitt K.V geöffnet wird. Im Beispiel von Figur 15 wird zunächst die Druckfeder 14 zusammengedrückt, nämlich dadurch, dass die Hülse 20 gegen die Hülse 10 drückt und die Hülse 10 die Druckfeder 14 zusammenschiebt. Der verdampferseitige Kanalabschnitt K.V wird geöffnet, während die Druckfeder 26 noch entspannt ist und ein Abstand zwischen der Hülse 25 und dem Kopf 33 auftritt und daher der behälterseitige Kanalabschnitt K.B noch gesperrt ist.

Wenn der Adapter 1100 aus der in Figur 15 gezeigten Position weiter auf den Anschlussabschnitt 1000 zu verschoben wird, so erreicht die Hülse 25 den Kopf 33, die Druckfeder 26 wird zusammengeschoben, und der behälterseitige Kanalabschnitt K.B wird geöffnet. Der behälterseitige Kanalabschnitt K.B und der verdampferseitige Kanalabschnitt K.V bilden nunmehr zusammen einen durchgehenden Kanal und realisieren eine Fluidverbindung von dem Narkosemittel-Behälter 400 in den Narkosemittel-Tank 304 des Narkosemittel-Verdampfers 300. Dank der Schwerkraft fließt das flüssige Narkosemittel durch diesen Kanal schräg nach unten in den Narkosemittel-Tank 304. Im Ausführungsbeispiel fließt nur dann flüssiges Narkosemittel durch den Kanal in den Narkosemittel-Tank 304, wenn und solange ein Benutzer den Narkosemittel-Behälter 400 gegen die Kraft der beiden Federn 14 und 26 in dem Durchlass-Zustand relativ zu dem Narkosemittel-Verdampfer 300 hält. Dadurch wird sichergestellt, dass während des Flusses von Narkosemittel stets ein Benutzer in der Nähe ist.

In der gezeigten Ausgestaltung wird ein einziger Kanal zwischen dem Narkosemittel-Behälter 400 und dem Narkosemittel-Verdampfer 300 bereitgestellt. Einerseits fließt flüssiges Narkosemittel schräg nach unten durch diesen Kanal. Andererseits entweicht Gas mit verdampftem Narkosemittel aus dem Narkosemittel-Tank 304 durch denselben Kanal schräg nach oben. Das trichterförmige Segment K.V3 im Fuß 15 sammelt und zentriert aufsteigendes Gas und leitet dieses Gas in das Segment K.V2. Dadurch wird die Gefahr reduziert, dass Gas aus dem Narkosemittel-Tank 304 in die Umgebung entweicht.

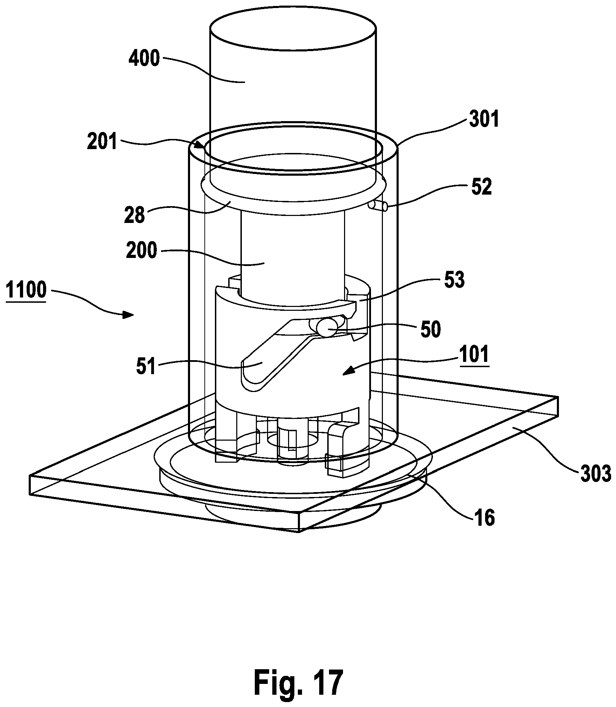

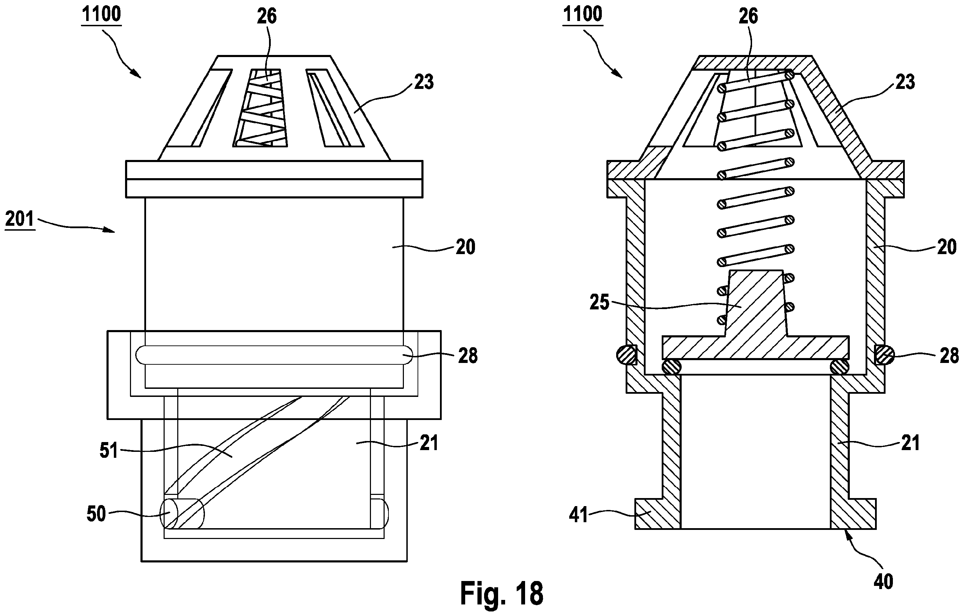

Figur 17 zeigt in einer Übersichtsdarstellung eine Bajonettverbindung zwischen dem Adapter 1100 und dem Anschlussabschnitt 1000. Figur 18 zeigt eine Detaildarstellung dieser Bajonettverbindung. Ein Vorsprung 50 an der größeren Hülse 10 des äußeren verdampferseitigen Bauteils 101 greift in eine korrespondierende schräg angeordnete Aussparung 51 an der größeren Hülse 20 des äußeren behälterseitigen Bauteils 201 ein. Um den Adapter 1100 mit dem Anschlussabschnitt 1000 zu verbinden, wird der Vorsprung 50 durch einen Schlitz 53 im äußeren verdampferseitigen Bauteil 101 hindurch geschoben, und anschließend wird der Adapter 1100 mitsamt dem Vorsprung 50 gedreht, bis der Vorsprung 50 das Ende der Aussparung 51 erreicht. Durch eine umgekehrte Bewegung wird der Adapter 1100 wieder vom Anschlussabschnitt 1000 entfernt. In Figur 17 ist außerdem ein optionales Ventil 52 zu sehen. Durch dieses Ventil 52 kann Gas aus dem Narkosemittel-Tank 301 entweichen, während der Narkosemittel-Tank 301 mit einem Narkosemittel befüllt wird.

**BEZUGSZEICHENLISTE**

| | |
|---|---|
| 1 | Kegelstumpf des Kopfs 33 des inneren verdampferseitigen Bauteils 100, dessen Oberfläche stellt die Kontaktfläche K.1 bereit |
| 2 | Scheibe des Kopfs 33 des inneren verdampferseitigen Bauteils 100, liegt am Kegelstumpf 1 an |
| 3 | innen hohler Hals des inneren verdampferseitigen Bauteils 100, trägt den Kopf 33 |
| 4 | umlaufender Dichtring, an der Scheibe 2 montiert, liegt an der Kontaktfläche K.8 an |
| 5 | Scheibe um den Hals 3, verschließt den im Inneren des Stellmittels 8 angeordneten Teil des verdampferseitigen Kanalabschnitts K.V |
| 6 | umlaufende Scheibe im Inneren des Stellmittels 8, fest mit dem Stellmittel 8 verbunden |
| 7 | Röhre des inneren verdampferseitigen Bauteils 100, von den Hülsen 10 und 11 umgeben, nimmt den verdampferseitigen Kanalabschnitt K.V2 auf |
| 8 | Stellmittel, mit dem Kragen 9 verbunden, gehört zum äußeren verdampferseitigen Bauteil 101, dessen Oberfläche stellt die Kontaktfläche K.8 bereit |
| 9 | Kragen des äußeren verdampferseitigen Bauteils 101, verbindet das Stellmittel 8 mit der größeren Hülse 10 |
| 10 | größere Hülse, mit dem Kragen 9 verbunden, gehört zum äußeren verdampferseitigen Bauteil |
| 11 | kleinere Hülse, fest mit der größeren Hülse 10 verbunden, gehört zum äußeren verdampferseitigen Bauteil 101 |
| 12 | Kragen des inneren verdampferseitigen Bauteils 100, verbindet den Hals 3 mit der Röhre 7, hat die Außenwand 36 |
| 13 | umlaufender Vorsprung des Stellmittels 8, umschließt die Kontaktfläche K.8 |
| 14 | verdampferseitiges Federelement, stützt sich an der Scheibe 15 ab, drückt gegen die größere Hülse 10, drückt das äußere verdampferseitige Bauteil 101 in die Sperr-Endposition |
| 15 | überstehende Scheibe am Fuß des inneren verdampferseitigen Bauteils 100, fest mit der Röhre 7 verbunden, umfasst eine Rinne für den Dichtring 16 |
| 16 | Dichtring, in einer Rinne der Scheibe 15 aufgenommen |
| 17 | kreisringförmige Aussparung in der Kontaktfläche K.8 |
| 18 | Dichtscheibe außen an der Röhre 7 |
| 19 | konusförmige Innenwand des Stellmittels 8, hat dann, wenn das Stellmittel 8 in der Freigabe-Endposition ist, den kleinstmöglichen Abstand zu der Außenwand 36 des Kragens 12 |
| 20 | größere Hülse des äußeren behälterseitigen Bauteils 201 |
| 21 | kleinere Hülse des äußeren behälterseitigen Bauteils 201 |
| 22 | Dichtscheibe um die größere Hülse 20 herum, in eine Öffnung des Narkosemittel-Behälters 400 aufgenommen, umfasst eine Rinne für den Dichtring 54 |
| 23 | Abstützelement des äußeren behälterseitigen Bauteils 201, fest mit der größeren Hülse 20 verbunden, ist bevorzugt domförmig oder zylindrisch |
| 24 | Röhre des inneren behälterseitigen Bauteils 200, am Abstützelement 23 befestigt, vom Federelement 26 umgeben |
| 25 | trichterförmige Hülse des inneren behälterseitigen Bauteils 200, nimmt den Kopf 33 auf, dessen Oberfläche stellt das Kontaktprofil K.25 bereit |
| 26 | behälterseitiges Federelement, stützt sich am Abstützelement 23 ab, drückt das innere behälterseitige Bauteil 200 in die Sperr-Endposition |
| 27 | Dichtring des inneren behälterseitigen Bauteils 200, an der trichterförmigen Hülse 25 befestigt |
| 28 | Dichtring des äußeren behälterseitigen Bauteils 201, an der Halterung 31 befestigt, gehört zum behälterseitigen Kontaktprofil |
| 29 | Kragen zwischen der größeren Hülse 20 und der kleineren Hülse 21 |
| 30 | umlaufende Scheibe, mit der größeren Hülse 20 verbunden, begrenzt die Bewegung des inneren behälterseitigen Bauteils 200 vom Narkosemittel-Behälter 400 weg |
| 31 | Halterung für den Dichtring 28, innen an der kleineren Hülse 21 befestigt, gehört zum behälterseitigen Kontaktprofil |
| 32 | Halterung für den Dichtring 27, außen an der trichterförmigen Hülse 25 befestigt |
| 33 | Kopf des inneren verdampferseitigen Bauteils 100, umfasst den Kegelstumpf 1 und die Scheibe 2, ist auf dem Hals 3 montiert |
| 34 | Röhre des inneren behälterseitigen Bauteils 200, an der trichterförmigen Hülse 25 befestigt |
| 35 | zylinderförmige Innenwand des inneren verdampferseitigen Bauteils 100 |
| 36 | Außenwand des Kragens 12, hat dann, wenn das Stellmittel 8 in der Freigabe-Endposition ist, den kleinstmöglichen Abstand zu der Innenwand 19 |
| 37 | Übergangsfläche zwischen der größeren Hülse 10 und der kleineren Hülse 11 |
| 38 | Gleitringe außen an der Röhre 7 |
| 40 | umlaufende Kontaktfläche an der kleineren Hülse 21 |
| 41 | umlaufender Vorsprung an der kleineren Hülse 21, grenzt an die umlaufende Kontaktfläche 40 an |
| 50 | Vorsprung für den Bajonettverbindung, an dem äußeren verdampferseitigen Bauteil 101 angeordnet, greift in die Aussparung 51 an |
| 51 | Aussparung für die Bajonettverbindung, an dem äußeren behälterseitigen Bauteil 201 angeordnet, nimmt den Vorsprung 50 auf |
| 52 | Ventil an dem äußeren behälterseitigen Bauteil 201 |
| 53 | Schlitz in der größeren Hülse 10 |
| 54 | Dichtring, in einer Rinne der Dichtscheibe 22 aufgenommen |
| 60 | Schrauben, welche das äußere verdampferseitige Bauteil 101 im Gehäuse 301 halten |
| 100 | inneres verdampferseitiges Bauteil |
| 101 | äußeres verdampferseitiges Bauteil |
| 200 | inneres behälterseitiges Bauteil |
| 201 | äußeres behälterseitiges Bauteil |
| 300 | Narkosemittel-Verdampfer, umfasst das Gehäuse 301, in welches der Anschlussabschnitt 1000 eingesetzt ist, sowie den Narkosemittel-Tank 304 |
| 301 | Gehäuse, welches den Anschlussabschnitt 1000 und den Adapter 1100 aufnimmt, gehört zum Narkosemittel-Verdampfer 300, trägt die Schnapphalterung 302 |
| 302 | Schnapphalterung an der Innenwand des Gehäuses 301, hält das äußere behälterseitige Bauteil 201 |
| 303 | Wand des Narkosemittel-Verdampfers 300, nimmt das Gehäuse 301 auf |
| 304 | Narkosemittel-Tank des Narkosemittel-Verdampfers 300 |
| 400 | Narkosemittel-Behälter, umfasst die Wand 401, in welche der Adapter 1100 eingesetzt ist |
| 401 | Wand des Narkosemittel-Behälters 400, in das der Adapter 1100 eingesetzt ist |
| 1000 | Anschlussabschnitt des Narkosemittel-Verdampfers 300, umfasst das innere verdampferseitige Bauteil 100, das äußere verdampferseitige Bauteil 101 und das verdampferseitige Federelement 14 |
| 1100 | Adapter des Narkosemittel-Behälters 400, umfasst das innere behälterseitige Bauteil 200, das äußere behälterseitige Bauteil 201 und das behälterseitige Federelement 26 |
| A | übereinstimmende Symmetrieachsen des Anschlussabschnitts 1000 und des Adapters 1100 |
| K.1 | Kontaktfläche an der Spitze des Kegelstumpfs 1, gehört zur verdampferseitigen Kontaktfläche, kommt in Kontakt mit der Kontaktfläche K.25 |
| K.8 | Kontaktfläche an der Spitze des Stellmittel 8, gehört zur verdampferseitigen Kontaktfläche, kommt in Kontakt mit dem Dichtring 28 und / oder der Halterung 31 |
| K.25 | Kontaktfläche im Inneren des trichterförmigen Segment 25, gehört zur behälterseitigen Kontaktfläche, kommt in Kontakt mit der Kontaktfläche K.1 |
| K.B1 | im Inneren der kleineren Hülse 21 angeordnetes Segment des behälterseitigen Kanalabschnitts K.B |
| K.B2 | im Inneren der größeren Hülse 20 angeordnetes Segment des behälterseitigen Kanalabschnitts K.B |
| K.B3 | im Inneren des domförmigen Elements 23 angeordnetes Segment des behälterseitigen Kanalabschnitts K.B |
| K.V1 | im Inneren des Stellmittels 8 angeordnetes Segment des verdampferseitigen Kanalabschnitts K.B |
| K.V2 | durch die Röhre 7 geführtes Segment des verdampferseitigen Kanalabschnitts K.V |
| K.V3 | trichterförmiges Segment des verdampferseitigen Kanalabschnitts K.V, im Inneren der Scheibe 15 angeordnet |
| Ö1 | Öffnungen in der Scheibe 6 |
| Ö2 | Öffnungen im Kragen 12 |
| Zw | Zwischenraum zwischen dem Gehäuse 301 und dem äußeren verdampferseitigen Bauteil 101, in den der Adapter 1100 eingreift |

## Patentansprüche

1. Verbindungs-Anordnung zum zeitweisen fluiddichten Verbinden eines Narkosemittel-Behälters (400) mit einem Narkosemittel-Verdampfer (300),
wobei die Verbindungs-Anordnung
- einen Anschlussabschnitt (1000), der mit dem Narkosemittel-Verdampfer (300) verbunden oder verbindbar ist, und
- einen Adapter (1100), der mit dem Narkosemittel-Behälter (400) verbunden oder verbindbar ist,
umfasst,
wobei der Anschlussabschnitt (1000)
- ein inneres verdampferseitiges Bauteil (100) und
- ein hohles äußeres verdampferseitiges Bauteil (101)
umfasst,
wobei das innere verdampferseitige Bauteil (100) durch das äußere verdampferseitige Bauteil (101) hindurch geführt ist,
wobei der Adapter (1100) sich lösbar mit dem Anschlussabschnitt (1000) verbinden lässt,
wobei der Anschlussabschnitt (1000) einen verdampferseitigen Kanalabschnitt (K.V) umfasst,
wobei der verdampferseitige Kanalabschnitt (K.V) durch das innere verdampferseitige Bauteil (100) hindurch geführt ist,
wobei der Adapter (1100) einen behälterseitigen Kanalabschnitt (K.B) umfasst und
wobei dann, wenn der Adapter (1100) mit dem Anschlussabschnitt (1000) verbunden ist,
- die beiden Kanalabschnitte (K.B, K.V) einen durchgehenden Kanal bilden, mit dessen Hilfe eine Fluidverbindung zwischen dem Narkosemittel-Behälter (400) und dem Narkosemittel-Verdampfer (300) hergestellt oder herstellbar ist,
- derjenige Bereich der Oberfläche des Anschlussabschnitts (1000), der zum Adapter (1100) hin zeigt, von einem verdampferseitigen Kontaktprofil (K.1, K.8) gebildet wird,
- derjenige Bereich der Oberfläche des Adapters (1100), der zum Anschlussabschnitt (1000) hin zeigt, von einem behälterseitigen Kontaktprofil (K.25, 28, 31) gebildet wird,
- die beiden Kontaktprofile (K.1, K.8, K.25, 28, 31) ohne Zwischenraum aneinander anliegen,
- das eine Kontaktprofil (K.1, K.8) einen Vorsprung (1) und das andere Kontaktprofil (K.25, 28, 31) eine korrespondierende Aussparung (25) aufweist und
- der Vorsprung (1) formschlüssig in die Aussparung (25) eingreift.

2. Verbindungs-Anordnung nach Anspruch 1,
**dadurch gekennzeichnet, dass**
das äußere verdampferseitige Bauteil (101) relativ zum inneren verdampferseitigen Bauteil (100) beweglich ist,
wobei das äußere verdampferseitige Bauteil (101) ein Stellmittel (8) umfasst,
wobei das Stellmittel (8) relativ zum inneren verdampferseitigen Bauteil (100)
- zwischen einer Sperr-Endposition, in der das Stellmittel (8) den verdampferseitigen Kanalabschnitt (K.V) verschließt, und
- einer Freigabe-Endposition, in der das Stellmittel (8) den verdampferseitigen Kanalabschnitt (K.V) freigibt,
beweglich ist.

3. Verbindungs-Anordnung nach Anspruch 2,
**dadurch gekennzeichnet, dass**
das Stellmittel (8) hohl ist und
das innere verdampferseitige Bauteil (100) durch das Stellmittel (8) hindurch geführt ist.

4. Verbindungs-Anordnung nach einen der Ansprüche 2 oder 3,
**dadurch gekennzeichnet, dass**
das innere verdampferseitige Bauteil (100) einen Kopf (33) und eine Röhre (7) umfasst,
wobei der Kopf (33)
- auf der Röhre (7) befestigt ist,
- den Vorsprung (1) des einen Kontaktprofils (K.1, K.8) bereitstellt und
- einen größeren maximalen Durchmesser als die Röhre (7) aufweist,
wobei der verdampferseitige Kanalabschnitt (K.V) durch die Röhre (7) hindurch geführt ist und
wobei das Stellmittel (8) in der Sperr-Endposition an dem Kopf (33) anliegt.

5. Verbindungs-Anordnung nach einen der vorhergehenden Ansprüche,
**dadurch gekennzeichnet, dass**
der Anschlussabschnitt (1000) ein Gehäuse (301) umfasst, welches das äußere verdampferseitige Bauteil (101) umgibt,
wobei dann, wenn der Adapter (1100) mit dem Anschlussabschnitt (1000) verbunden ist,
der Adapter (1100) in einen Zwischenraum (Zw) zwischen dem Gehäuse (301) und dem äußeren verdampferseitigen Bauteil (101) eingreift.

6. Verbindungs-Anordnung nach einen der vorhergehenden Ansprüche,
**dadurch gekennzeichnet, dass**
das Stellmittel (8) ein zum inneren verdampferseitigen Bauteil (100) hin zeigendes Innenprofil (19) aufweist und
das innere verdampferseitige Bauteil (100) ein Segment (7, 12) mit einem zum Stellmittel (8) hin zeigenden Außenprofil (36) aufweist,
wobei der verdampferseitige Kanalabschnitt (K.V) durch dieses Segment (7, 12) hindurch geführt ist.

7. Verbindungs-Anordnung nach einem der vorhergehenden Ansprüche,
**dadurch gekennzeichnet, dass**
der Adapter (1100)
- ein hohles äußeres behälterseitiges Bauteil (201) und
- ein inneres behälterseitiges Bauteil (200)
umfasst,
wobei das innere behälterseitige Bauteil (200) relativ zum äußeren behälterseitigen Bauteil (201) beweglich ist und
wobei der behälterseitige Kanalabschnitt (K.B) zwischen dem inneren behälterseitigen Bauteil (200) und dem äußeren behälterseitigen Bauteil (201) hindurch geführt ist.

8. Verbindungs-Anordnung nach Anspruch 7,
**dadurch gekennzeichnet, dass**
das innere behälterseitige Bauteil (200) relativ zum äußeren behälterseitigen Bauteil (201) zwischen
- einer Sperr-Endposition, in welcher das innere behälterseitige Bauteil (200) den behälterseitigen Kanalabschnitt (K.B) versperrt, und
- einer Freigabe-Endposition, in welcher das innere behälterseitige Bauteil (200) den behälterseitigen Kanalabschnitt (K.B) freigibt,
beweglich ist.

9. Verbindungs-Anordnung nach Anspruch 7 oder Anspruch 8,
**dadurch gekennzeichnet, dass**
der Adapter (1100) ein behälterseitiges Federelement (26) umfasst und
das äußere behälterseitige Bauteil (201) ein Abstützelement (23) mit mindestens einer Aussparung umfasst,
wobei das behälterseitige Federelement (26)
- sich am äußeren behälterseitigen Bauteil (201) abstützt und
- bestrebt ist, das innere behälterseitige Bauteil (200) auf den Anschlussabschnitt (1000) zuzubewegen,
wobei das Abstützelement (23) zum Narkosemittel-Behälter (400) hin zeigt,
wobei der behälterseitige Kanalabschnitt (K.B) durch die oder mindestens einer Aussparung im Abstützelement (23) hindurch geführt ist und
wobei das behälterseitige Federelement (26) sich am Abstützelement (23) abstützt.

10. Verbindungs-Anordnung nach einem der Ansprüche 7 bis 9,
**dadurch gekennzeichnet, dass**
dann, wenn der Adapter (1100) mit dem Anschlussabschnitt (1000) verbunden ist,
- das äußere behälterseitige Bauteil (201) ein zum Adapter (1100) zeigendes vorderes Segment (8, 13) des äußeren verdampferseitigen Bauteils (101) wenigstens teilweise umgibt und
- das innere behälterseitige Bauteil (200) ein zum Adapter (1100) zeigendes vorderes Segment (33) des inneren verdampferseitigen Bauteils (100) wenigstens teilweise umgibt.

11. Verbindungs-Anordnung nach einem der vorhergehenden Ansprüche,
**dadurch gekennzeichnet, dass**
der verdampferseitige Kanalabschnitt (K.V) an einem dem Narkosemittel-Verdampfer (300) zugewandten Ende einen trichterförmigen Abschnitt (K.V3) aufweist,
dessen Durchmesser sich in Richtung des Narkosemittel-Verdampfers (300) vergrößert.

12. Verbindungs-Anordnung nach einem der vorhergehenden Ansprüche,
**dadurch gekennzeichnet, dass**
der Adapter (1100) relativ zu dem Anschlussabschnitt (1000)
- in einen Koppel-Zustand bringbar ist, in dem der Adapter (1100) den Anschlussabschnitt (1000) berührt und der Adapter (1100) in den Anschlussabschnitt (1000) oder der Anschlussabschnitt (1000) in den Adapter (1100) eingreift, mindestens ein Kanalabschnitt (K.B) aber noch gesperrt ist, und
- aus dem Koppel-Zustand in einen Durchlass-Zustand bringbar ist, in dem die beiden Kontaktprofile (K.1, K.8, K.25, 28, 31) ohne Zwischenraum aneinander anliegen und die beiden Kanalabschnitte (K.B, K.V) geöffnet sind und den durchgehenden Kanal bilden.

13. System umfassend
einen Narkosemittel-Verdampfer (300) mit einem Anschlussabschnitt (1000) und
einen Narkosemittel-Behälter (400) mit einem Adapter (1100),
wobei der Anschlussabschnitt (1000) und der Adapter (1100) zusammen eine Verbindungs-Anordnung nach einem der vorhergehenden Ansprüche bereitstellen.

14. Anschlussabschnitt (1000) für einen Narkosemittel-Verdampfer (300),
wobei der Anschlussabschnitt (1000) sich fluiddicht mit einem Adapter (1100) eines Narkosemittel-Behälters (400) verbinden lässt,
wobei der Anschlussabschnitt (1000)
- ein inneres verdampferseitiges Bauteil (100) und
- ein hohles äußeres verdampferseitiges Bauteil (101)
umfasst,
wobei der Anschlussabschnitt (1000)
- einen verdampferseitigen Kanalabschnitt (K.V) und
- ein verdampferseitiges Kontaktprofil (K.1, K.8) mit einem Vorsprung (1) umfasst,
wobei das innere verdampferseitige Bauteil (100) durch das äußere verdampferseitige Bauteil (101) hindurch geführt ist,
wobei der verdampferseitige Kanalabschnitt (K.V) durch das innere verdampferseitige Bauteil (100) hindurch geführt ist,
wobei der verdampferseitige Kanalabschnitt (K.V) dazu ausgestaltet ist, zusammen mit einem korrespondierenden behälterseitigen Kanalabschnitt (K.B) einen durchgehenden Kanal zu bilden, wenn der Anschlussabschnitt (1000) mit dem Adapter (1100) verbunden ist,
wobei das verdampferseitige Kontaktprofil (K.1, K.8) dazu ausgestaltet ist, denjenigen Bereich der Oberfläche des Anschlussabschnitts (1000) zu bilden, der zum Adapter (1100) hin zeigt, wenn der Adapter (1100) mit dem Anschlussabschnitt (1000) verbunden ist, und
wobei der Anschlussabschnitt (1000) so ausgestaltet ist, dass dann, wenn der Anschlussabschnitt (1000) mit dem Adapter (1100) verbunden ist,
- das verdampferseitige Kontaktprofil (K.1, K.8) ohne Zwischenraum an einem korrespondierenden behälterseitigen Kontaktprofil (K.25, 28, 31) anliegt und
- der Vorsprung (1) formschlüssig in eine korrespondierende Aussparung (25) des behälterseitigen Kontaktprofils (K.25, 28, 31) eingreift.

15. Narkosemittel-Verdampfer mit einem Anschlussabschnitt (1000) nach Anspruch 14.

## Claims

1. Connection arrangement for temporary fluid-tight connection of an anaesthetic container (400) to an anaesthetic vaporizer (300),
wherein the connection arrangement comprises
- a connection section (1000) which is connected or connectable to the anaesthetic vaporizer (300), and
- an adaptor (1100) which is connected or connectable to the anaesthetic container (400),
wherein the connection section (1000) comprises
- an inner vaporizer-side component (100) and
- a hollow outer vaporizer-side component (101),
wherein the inner vaporizer-side component (100) is guided through the outer vaporizer-side component (101),
wherein the adaptor (1100) is able to be releasably connected to the connection section (1000),
wherein the connection section (1000) comprises a vaporizer-side channel section (K.V),
wherein the vaporizer-side channel section (K.V) is guided through the inner vaporizer-side component (100),
wherein the adaptor (1100) comprises a container-side channel section (K.B), and
wherein, when the adaptor (1100) is connected to the connection section (1000),
- the two channel sections (K.B, K.V) form a continuous channel, with the aid of which a fluid connection between the anaesthetic container (400) and the anaesthetic vaporizer (300) is or is able to be established,
- that region of the surface of the connection section (1000) which points towards the adaptor (1100) is formed by a vaporizer-side contact profile (K.1, K.8),
- that region of the surface of the adaptor (1100) which points towards the connection section (1000) is formed by a container-side contact profile (K.25, 28, 31),
- the two contact profiles (K.1, K.8, K.25, 28, 31) bear against one another without any space between them,
- one contact profile (K.1, K.8) has a projection (1) and the other contact profile (K.25, 28, 31) has a corresponding cutout (25), and
- the projection (1) engages into the cutout (25) in a form-fitting manner.

2. Connection arrangement according to Claim 1,
**characterized in that**
the outer vaporizer-side component (101) is movable relative to the inner vaporizer-side component (100),
wherein the outer vaporizer-side component (101) comprises an actuating means (8),
wherein the actuating means (8) is movable relative to the inner vaporizer-side component (100)
- between a blocking end position, in which the actuating means (8) closes off the vaporizer-side channel section (K.V), and
- a release end position, in which the actuating means (8) opens up the vaporizer-side channel section (K.V).

3. Connection arrangement according to Claim 2,
**characterized in that**
the actuating means (8) is hollow, and
the inner vaporizer-side component (100) is guided through the actuating means (8).

4. Connection arrangement according to either of Claims 2 and 3,
**characterized in that**
the inner vaporizer-side component (100) comprises a head (33) and a tube (7),
wherein the head (33)
- is fastened to the tube (7),
- provides the projection (1) of one contact profile (K.1, K.8), and
- has a larger maximum diameter than the tube (7),
wherein the vaporizer-side channel section (K.V) is guided through the tube (7), and
wherein the actuating means (8) bears against the head (33) in the blocking end position.

5. Connection arrangement according to one of the preceding claims,
**characterized in that**
the connection section (1000) comprises a housing (301) which surrounds the outer vaporizer-side component (101),
wherein, when the adaptor (1100) is connected to the connection section (1000),
the adaptor (1100) engages into an intermediate space (Zw) between the housing (301) and the outer vaporizer-side component (101).

6. Connection arrangement according to one of the preceding claims,
**characterized in that**
the actuating means (8) has an inner profile (19) which points towards the inner vaporizer-side component (100), and
the inner vaporizer-side component (100) has a segment (7, 12) with an outer profile (36) which points towards the actuating means (8),
wherein the vaporizer-side channel section (K.V) is guided through said segment (7, 12).

7. Connection arrangement according to one of the preceding claims,
**characterized in that**
the adaptor (1000) comprises
- a hollow outer container-side component (201), and
- an inner container-side component (200),
wherein the inner container-side component (200) is movable relative to the outer container-side component (201), and
wherein the container-side channel section (K.B) is guided between the inner container-side component (200) and the outer container-side component (201).

8. Connection arrangement according to Claim 7,
**characterized in that**
the inner container-side component (200) is movable relative to the outer container-side component (201) between
- a blocking end position, in which the inner container-side component (200) closes off the container-side channel section (K.B), and
- a release end position, in which the inner container-side component (200) opens up the container-side channel section (K.B).

9. Connection arrangement according to Claim 7 or Claim 8,
**characterized in that**
the adaptor (1100) comprises a container-side spring element (26), and the outer container-side component (201) comprises a support element (23) with at least one cutout,
wherein the container-side spring element (26)
- is supported against the outer container-side component (201), and
- strives to move the inner container-side component (200) towards the connection section (1000),
wherein the support element (23) points towards the anaesthetic container (400),
wherein the container-side channel section (K.B) is guided through the or at least one cutout in the support element (23), and
wherein the container-side spring element (26) is supported against the support element (23).

10. Connection arrangement according to one of Claims 7 to 9,
**characterized in that**,
when the adaptor (1100) is connected to the connection section (1000),
- the outer container-side component (201) at least partially surrounds a front segment (8, 13), pointing to the adaptor (1100), of the outer vaporizer-side component (101), and
- the inner container-side component (200) at least partially surrounds a front segment (33), pointing to the adaptor (1100), of the inner vaporizer-side component (100).

11. Connection arrangement according to one of the preceding claims,
**characterized in that**,
on an end facing towards the anaesthetic vaporizer (300), the vaporizer-side channel section (K.V) has a funnel-shaped section (K.V3),
the diameter of which increases in the direction of the anaesthetic vaporizer (300).

12. Connection arrangement according to one of the preceding claims,
**characterized in that**,
relative to the connection section (1000), the adaptor (1100)
- is able to be brought into a coupled state, in which the adaptor (1100) makes contact with the connection section (1000) and the adaptor (1100) engages into the connection section (1000) or the connection section (1000) engages into the adaptor (1100), at least one channel section (K.B) still being blocked however, and
- is able to be brought from the coupled state into a pass-through state, in which the two contact profiles (K.1, K.8, K.25, 28, 31) bear against one another without any space between them and the two channel sections (K.B, K.V) are open and form the continuous channel.

13. System comprising
an anaesthetic vaporizer (300) having a connection section (1000) and an anaesthetic container (400) having an adaptor (1100),
wherein the connection section (1000) and the adaptor (1100) together provide a connection arrangement according to one of the preceding claims.

14. Connection section (1000) for an anaesthetic vaporizer (300),
wherein the connection section (1000) is able to be connected in a fluid-tight manner to an adaptor (1100) of an anaesthetic container (400),
wherein the connection section (1000) comprises
- an inner vaporizer-side component (100) and
- a hollow outer vaporizer-side component (101),
wherein the connection section (1000) comprises
- a vaporizer-side channel section (K.V) and
- a vaporizer-side contact profile (K.1, K.8) with a projection (1),
wherein the inner vaporizer-side component (100) is guided through the outer vaporizer-side component (101),
wherein the vaporizer-side channel section (K.V) is guided through the inner vaporizer-side component (100),
wherein the vaporizer-side channel section (K.V) is configured such that, when the connection section (1000) is connected to the adaptor (1100), it forms, together with a corresponding container-side channel section (K.B), a continuous channel, and
wherein the vaporizer-side contact profile (K.1, K.8) is configured such that, when the adaptor (1100) is connected to the connection section (1000), it forms that region of the surface of the connection section (1000) which points towards the adaptor (1100), and
wherein the connection section (1000) is configured in such a way that, when the connection section (1000) is connected to the adaptor (1100),
- the vaporizer-side contact profile (K.1, K.8) bears against a corresponding container-side contact profile (K.25, 28, 31) without any space between them, and
- the projection (1) engages into a corresponding cutout (25) of the container-side contact profile (K.25, 28, 31) in a form-fitting manner.

15. Anaesthetic vaporizer having a connection section (1000) according to Claim 14.

## Revendications

1. Ensemble de liaison destiné à relier par intermittence, avec étanchéité aux fluides, un récipient (400) d'agent anesthésique à un évaporateur (300) d'agent anesthésique,
lequel ensemble de liaison inclut
- une zone de raccordement (1000) reliée, ou pouvant être reliée audit évaporateur (300) d'agent anesthésique, et
- un adaptateur (1100) relié, ou pouvant être relié audit récipient (400) d'agent anesthésique,
ladite zone de raccordement (1000) incluant
- une pièce structurelle intérieure (100) située côté évaporateur et
- une pièce structurelle extérieure (101) creuse, située côté évaporateur,
ladite pièce structurelle intérieure (100), située côté évaporateur, parcourant l'intégralité de ladite pièce structurelle extérieure (101) située côté évaporateur, l'adaptateur (1100) pouvant être relié amoviblement à la zone de raccordement (1000),
ladite zone de raccordement (1000) comportant un tronçon de canal (K.V) situé côté évaporateur,
lequel tronçon de canal (K.V), situé côté évaporateur, parcourt l'intégralité de la pièce structurelle intérieure (100) située côté évaporateur,
sachant que l'adaptateur (1100) comporte un tronçon de canal (K.B) situé côté récipient et que, lorsque ledit adaptateur (1100) est relié à la zone de raccordement (1000),
- les deux tronçons de canal (K.B, K.V) forment un canal ininterrompu à l'aide duquel une liaison fluidique est, ou peut être instaurée entre le récipient (400) d'agent anesthésique et l'évaporateur (300) d'agent anesthésique,
- la région de la surface de ladite zone de raccordement (1000), qui pointe en direction dudit adaptateur (1100), est constituée d'un profil de contact (K.1, K.8) situé côté évaporateur,
- la région de la surface dudit adaptateur (1100), qui pointe en direction de ladite zone de raccordement (1000), est constituée d'un profil de contact (K.25, 28, 31) situé côté récipient,
- les deux profils de contact (K.1, K.8, K.25, 28, 31) portent l'un contre l'autre sans espace interstitiel,
- l'un (K.1, K.8) desdits profils de contact étant doté d'une protubérance (1), et l'autre profil de contact (K.25, 28, 31) étant pourvu d'un évidement (25) concordant, et
- ladite protubérance (1) pénétrant par complémentarité de formes dans ledit évidement (25).

2. Ensemble de liaison selon la revendication 1,
**caractérisé par le fait que**
la pièce structurelle extérieure (101), située côté évaporateur, est mobile par rapport à la pièce structurelle intérieure (100) située côté évaporateur,
ladite pièce structurelle extérieure (101), située côté évaporateur, incluant un moyen de réglage (8),
lequel moyen de réglage (8) est mobile, par rapport à ladite pièce structurelle intérieure (100) située côté évaporateur,
- entre un emplacement extrême de blocage, auquel ledit moyen de réglage (8) obture le tronçon de canal (K.V) situé côté évaporateur, et
- un emplacement extrême de libération auquel ledit moyen de réglage (8) libère ledit tronçon de canal (K.V) situé côté évaporateur.

3. Ensemble de liaison selon la revendication 2,
**caractérisé par le fait que**
le moyen de réglage (8) est creux et
la pièce structurelle intérieure (100), située côté évaporateur, parcourt l'intégralité dudit moyen de réglage (8).

4. Ensemble de liaison selon l'une des revendications 2 ou 3,
**caractérisé par le fait que**
la pièce structurelle intérieure (100), située côté évaporateur, comprend une tête (33) et une tubulure (7),
sachant que ladite tête (33)
- est fixée sur ladite tubulure (7),
- procure la protubérance (1) de l'un (K.1, K.8) des profils de contact et
- présente un diamètre maximal supérieur à celui de ladite tubulure (7),
le tronçon de canal (K.V), situé côté évaporateur, parcourant l'intégralité de ladite tubulure (7) et
le moyen de réglage (8) étant en applique sur ladite tête (33) à l'emplacement extrême de blocage.

5. Ensemble de liaison selon l'une des revendications précédentes, **caractérisé par le fait que**
la zone de raccordement (1000) inclut un boîtier (301) qui entoure la pièce structurelle extérieure (101) située côté évaporateur,
sachant que, lorsque l'adaptateur (1100) est relié à ladite zone de raccordement (1000),
ledit adaptateur (1100) pénètre dans un espace interstitiel (Zw) interposé entre ledit boîtier (301) et ladite pièce structurelle extérieure (101) située côté évaporateur.

6. Ensemble de liaison selon l'une des revendications précédentes,
**caractérisé par le fait que**
le moyen de réglage (8) est muni d'un profil interne (19) pointant en direction de la pièce structurelle intérieure (100) située côté évaporateur, et
ladite pièce structurelle intérieure (100), située côté évaporateur, est dotée d'un segment (7, 12) présentant un profil externe (36) qui pointe en direction dudit moyen de réglage (8),
le tronçon de canal (K.V), situé côté évaporateur, parcourant l'intégralité de ce segment (7, 12).

7. Ensemble de liaison selon l'une des revendications précédentes,
**caractérisé par le fait que**
l'adaptateur (1100) comprend
- une pièce structurelle extérieure (201) creuse, située côté récipient, et
- une pièce structurelle intérieure (200) située côté récipient,
sachant que ladite pièce structurelle intérieure (200), située côté récipient, est mobile par rapport à ladite pièce structurelle extérieure (201) située côté récipient et
sachant que le tronçon de canal (K.B), situé côté récipient, s'étend d'un trait entre ladite pièce structurelle intérieure (200), située côté récipient, et ladite pièce structurelle extérieure (201) située côté récipient.

8. Ensemble de liaison selon la revendication 7,
**caractérisé par le fait que**
la pièce structurelle intérieure (200) située côté récipient est mobile, par rapport à la pièce structurelle extérieure (201) située côté récipient, entre
- un emplacement extrême de blocage auquel ladite pièce structurelle intérieure (200), située côté récipient, obture le tronçon de canal (K.B) situé côté récipient, et
- un emplacement extrême de libération auquel ladite pièce structurelle intérieure (200), située côté récipient, libère ledit tronçon de canal (K.B) situé côté récipient.

9. Ensemble de liaison selon la revendication 7 ou la revendication 8,
**caractérisé par le fait que**
l'adaptateur (1100) inclut un élément élastique (26), situé côté récipient, et
la pièce structurelle extérieure (201), située côté récipient, inclut un élément d'appui (23) pourvu d'au moins un évidement,
sachant que ledit élément élastique (26), situé côté récipient,
- est en appui sur ladite pièce structurelle extérieure (201) située côté récipient et
- tend à mouvoir, en direction de la zone de raccordement (1000), la pièce structurelle intérieure (200) située côté récipient,
sachant que ledit élément d'appui (23) pointe en direction du récipient (400) d'agent anesthésique,
sachant que le tronçon de canal (K.B), situé côté récipient, franchit intégralement l'évidement, ou au moins un évidement pratiqué dans ledit élément d'appui (23), et sachant que ledit élément élastique (26), situé côté récipient, est en appui sur ledit élément d'appui (23).

10. Ensemble de liaison selon l'une des revendications 7 à 9,
**caractérisé par le fait que**,
lorsque l'adaptateur (1100) est relié à la zone de raccordement (1000),
- la pièce structurelle extérieure (201) située côté récipient entoure, au moins partiellement, un segment antérieur (8, 13) de la pièce structurelle extérieure (101) située côté évaporateur, qui est orienté vers ledit adaptateur (1100), et
- la pièce structurelle intérieure (200) située côté récipient entoure, au moins partiellement, un segment antérieur (33) de la pièce structurelle intérieure (100) située côté évaporateur, qui est orienté vers ledit adaptateur (1100).

11. Ensemble de liaison selon l'une des revendications précédentes,
**caractérisé par le fait que**
le tronçon de canal (K.V) situé côté évaporateur est pourvu, à une extrémité pointant vers ledit évaporateur (300) d'agent anesthésique, d'une région infundibuliforme (K.V3)
dont le diamètre croît en direction dudit évaporateur (300) d'agent anesthésique.

12. Ensemble de liaison selon l'une des revendications précédentes,
**caractérisé par le fait que**
l'adaptateur (1100) peut être amené, par rapport à la zone de raccordement (1000),
- à un état de couplage dans lequel ledit adaptateur (1100) est en contact avec ladite zone de raccordement (1000) et ledit adaptateur (1100) pénètre dans ladite zone de raccordement (1000), ou bien ladite zone de raccordement (1000) pénètre dans ledit adaptateur (1100), mais au moins un tronçon de canal (K.B) demeure toutefois bloqué,
et,
- à partir dudit état de couplage, à un état qui autorise un passage et dans lequel les deux profils de contact (K.1, K.8, K.25, 28, 31) portent l'un contre l'autre, sans espace interstitiel, et les deux tronçons de canal (K.B, K.V) sont ouverts et forment le canal ininterrompu.

13. Système comprenant
un évaporateur (300) d'agent anesthésique, muni d'une zone de raccordement (1000), et
un récipient (400) d'agent anesthésique, doté d'un adaptateur (1100),
ladite zone de raccordement (1000) et ledit adaptateur (1100) procurant, en association, un ensemble de liaison conforme à l'une des revendications précédentes.

14. Zone de raccordement (1000) dévolue à un évaporateur (300) d'agent anesthésique,
laquelle zone de raccordement (1000) peut être reliée, avec étanchéité aux fluides, à un adaptateur (1100) d'un récipient (400) d'agent anesthésique,
ladite zone de raccordement (1000) incluant
- une pièce structurelle intérieure (100) située côté évaporateur et
- une pièce structurelle extérieure (101) creuse, située côté évaporateur,
sachant que ladite zone de raccordement (1000) comprend
- un tronçon de canal (K.V) situé côté évaporateur et
- un profil de contact (K.1, K.8), situé côté évaporateur et nanti d'une protubérance (1),
ladite pièce structurelle intérieure (100), située côté évaporateur, parcourant l'intégralité de ladite pièce structurelle extérieure (101) située côté évaporateur, ledit tronçon de canal (K.V), situé côté évaporateur, parcourant l'intégralité de ladite pièce structurelle intérieure (100) située côté évaporateur,
sachant que ledit tronçon de canal (K.V) situé côté évaporateur est conçu pour former, lorsque ladite zone de raccordement (1000) est reliée audit adaptateur (1100), un canal ininterrompu en association avec un tronçon de canal (K. B) concordant, situé côté récipient,
ledit profil de contact (K.1, K.8) situé côté évaporateur étant conçu pour former, lorsque ledit adaptateur (1100) est relié à ladite zone de raccordement (1000), la région de la surface de ladite zone de raccordement (1000) qui pointe en direction dudit adaptateur (1100), et
la zone de raccordement (1000) étant conçue de façon telle que, lorsque ladite zone de raccordement (1000) est reliée audit adaptateur (1100),
- ledit profil de contact (K.1, K.8) situé côté évaporateur soit en applique, sans espace interstitiel, sur un profil de contact (K.25, 28, 31) concordant, situé côté récipient, et
- ladite protubérance (1) pénètre, par complémentarité de formes, dans un évidement concordant (25) dudit profil de contact (K.25, 28, 31) situé côté récipient.

15. Evaporateur d'agent anesthésique, muni d'une zone de raccordement (1000) conforme à la revendication 14.
